# EUROPEAN PATENT SPECIFICATION

(11) **EP 0 640 828 B1**
(45) Date of publication and mention of the grant of the patent: **10.05.2000**
(21) Application number: 94112728.4
(22) Date of filing: 16.08.1994
(51) Int. Cl.: G01N 21/64, C12Q 1/68

(54) **Monitoring multiple reactions simultaneously and analyzing same**
Gleichzeitige Kontrolle mehrfacher Reaktionen und Analyse derselben
Contrôle simultané de réactions multiples, et analyse de telles réactions

(30) Priority: 27.08.1993 US 113168; 05.07.1994 US 266061
(43) Date of publication of application: 01.03.1995
(73) Proprietor: F. Hoffmann-La Roche AG, 4002 Basel (CH)
(72) Inventor: Higuchi, Russell G., Alameda, california 94501 (US); Watson, Robert M., Berkeley, California 94703 (US)
(74) Representative: AMMANN PATENTANWAELTE AG BERN

(56) References cited:
- EP-A- 0 266 881
- EP-A- 0 512 334
- WO-A-92/05278
- US-A- 5 038 852
- PATENT ABSTRACTS OF JAPAN vol. 11, no. 49 (P-547) 14 February 1987 & JP-A-61 215 948 (FUJIREBIO) 25 September 1986
- PATENT ABSTRACTS OF JAPAN vol. 11, no. 313 (P-626) 13 October 1987 & JP-A-62 105 031 (FUJIREBIO) 15 May 1987
- PATENT ABSTRACTS OF JAPAN vol. 15, no. 331 (P-1241) 22 August 1991 & JP-A-03 122 552 (FUJIREBIO) 31 May 1989
- PATENT ABSTRACTS OF JAPAN vol. 16, no. 63 (C-0911) 18 February 1992 & JP-A-03 259 099 (TEIJIN) 19 November 1991
- PATENT ABSTRACTS OF JAPAN vol. 16, no. 190 (C-0937) 8 May 1992 & JP-A-04 027 399 (SHIONOGI) 30 January 1992
- PATENT ABSTRACTS OF JAPAN vol. 16, no. 308 (P-1381) 7 July 1992 & JP-A-04 084 751 (SHIMADZU) 18 March 1992

## Description

The present invention relates to detecting nucleic acid (DNA or RNA) amplification, and more particularly to real-time monitoring of multiple nucleic acid amplification reactions, simultaneously. The invention also relates to a method for using the accumulated data to quantitate the starting concentration of a target nucleic acid sequence provided in one or more of the mixtures and monitor the effect of amplification reaction conditions on the reaction kinetics.

Various methods for detecting nucleic acid amplification are known. A number of assays are known which quantitate the number of starting DNA templates in a polymerase chain reaction (PCR), for example. Some involve the measurement of PCR product at the end of temperature thermal cycling and relate this level to the starting DNA concentration. Such an "endpoint" analysis, as has been typically done using PCR, reveals the presence or absence of target DNA but generally does not provide a usable measure of the starting number of DNA targets.

Other assays involve the use of a competitor amplification product whose template is added at known concentration to the reaction mixture before thermal cycling. In competitor-product protocols, an aliquot of the amplification is examined by gel-electrophoresis. The relative amount of target-specific and competitor PCR product is measured; this ratio is used to calculate the starting number of target templates. The larger the ratio of target-specific product to competitor-specific product, the higher the starting DNA concentration.

In addition to requiring "downstream" processing, such as hybridization or gel electrophoresis, these other assays are more limited in dynamic range (i.e., sensitivity to a range of target nucleic acid concentrations). In competitor assays, the sensitivity to template concentration differences is compromised when either the target or added competitor DNA is greatly in excess of the other. The dynamic range of assays that measure the amount of end product can also be limited in that at the chosen number of cycles some reactions may have reached a "plateau" level of product. Differences in starting template levels in these reactions are therefore not well reflected. Furthermore, small differences in the measured amount of product result in widely varying estimates of the starting template concentration, leading to great inaccuracy due to variable reaction conditions, variations in sampling, or the presence of inhibitors.

Optimization of PCR conditions typically is accomplished by measuring the effect of different conditions on the final yield and specificity of PCR product. In order to obtain information throughout amplification, many replicate samples are placed in a thermal cycler so that each can be removed from the thermal cycler at a different temperature cycle. The removed tubes then are analyzed by gel electrophoresis as a function of cycle number. As is apparent from this description, such optimization is complex and time-consuming in that it requires numerous sample manipulation steps as well as downstream electrophoresis analysis.

Any assay intended for large-scale (e.g., clinical) use should not only be reliable, but should be simplified as much as possible in order to facilitate its automation. Thus, there is a need for an apparatus and method for collecting data indicative of nucleic acid amplification that can be used, for example, to quantitate sample starting concentrations and optimize reaction conditions, that provides reliable results and is suitable for automation.

The aim of the present invention is to provide a nucleic acid amplification detection method and apparatus which makes possible to monitor the amplification of multiple amplification reaction mixtures, simultaneously in real-time, and which thereby makes possible to overcome the problems and disadvantages of the prior art.

According to the invention this aim is achieved by providing an apparatus that is characterized in that it comprises:
a thermal cycler including a heat conducting member having multiple recesses formed therein; and
a sensor arranged for detecting light emitted from said recesses, simultaneously.

In a preferred embodiment the apparatus according to the invention it further comprises a light source optically coupled to said thermal cycler and arranged to distribute light over a portion of said heat conducting member having a plurality of said recesses formed therein.

In another preferred embodiment of the apparatus according to the invention said recesses of the heat conducting member of the thermal cycler are formed through a surface thereof for receiving reaction vessels containing a nucleic acid amplification reaction mixtures; and said sensor is an imaging device optically coupled to said heat conducting member for generating an image of said surface and reaction vessels when said vessels are disposed in said recesses of the heat conducting member.

In a further preferred embodiment of the apparatus according to the invention said heat conducting member of the thermal cycler has multiple recesses formed therein and adapted for receiving nucleic acid amplification reaction mixtures, and the apparatus further comprises
a housing positioned over said heat conducting member and coupled to said thermal cycler;
a light source arranged to emit light in said housing and toward said recesses; and
a dichroic mirror positioned in said housing and above said recesses, said dichroic mirror being transmissive to light having a first wavelength and reflective to light having a second wavelength that differs from said first wavelength; and
said sensor being arranged to receive light reflected from said second surface of said dichroic mirror.

In a further preferred embodiment of the apparatus according to the invention said heat conducting member of the thermal cycler has multiple recesses formed therein and adapted for receiving nucleic acid amplification reaction mixtures including a nucleic acid sequence and a fluorescent binding agent and the apparatus further comprises
a housing positioned over said heat conducting member and being coupled to said thermal cycler;
a light source arranged to emit light in said housing;
a sensor arranged in said housing; and
a dichroic mirror positioned in said housing and above said recesses, said dichroic mirror being transmissive to light having a wavelength corresponding to the wavelength of light generated by the excitation light source and reflective to light having a wavelength corresponding to the wavelength of fluorescence emitted from a nucleic acid amplification mixture, including a fluorescent binding agent, when that mixture is disposed in one of the recesses formed in the heat conducting member and exposed to light from the excitation light source, said mirror be oriented to form an optical path between said recesses and said sensor.

In one embodiment of the apparatus according to the invention, a CCD-camera detects the accumulation of double-stranded DNA (dsDNA) in each of multiple polymerase chain reactions, simultaneously, using the increase in the fluorescence of a detectable fluorescent dye initially introduced into each amplification reaction mixture. The fluorescence results from the fluorescent dye binding duplex DNA. This embodiment advantageously eliminates the need for fiber-optic leads and the accompanying problems associated with coupling the fiber-optics to the individual reaction mixtures.

According to another aspect of the invention, the optical system that moves excitation light from a source to the multiple reaction mixtures being amplified in a thermal cycler, which forms part of the apparatus, is configured to provide excitation light to the reaction mixtures in a uniform manner. That is, the optical system permits excitation light to be essentially uniformly distributed over the thermal cycler heat exchanger so that each amplification reaction mixture receives essentially the same amount of excitation light.

With this apparatus, the fluorescence data can be collected and used to quantitate the initial amount of target nucleic acid sequence. In a preferred method for quantitation, multiple amplification reaction mixtures are provided. One amplification reaction mixture has an unknown concentration of a specific nucleic acid sequence. The other reaction mixtures include the same specific nucleic acid sequence in differing but known concentrations. The amplification reaction mixtures of known and unknown nucleic acid concentration are thermally cycled in parallel for multiple cycles. The fluorescence emitted from the reaction mixtures is monitored in real time and the number of cycles necessary for each reaction mixture to fluoresce at a certain intensity value determined . The number of cycles necessary for the mixture of unknown nucleic acid concentration to reach that value is compared to the number of cycles necessary for the mixtures of known nucleic acid concentration to reach that value to obtain the initial quantity of said specific nucleic acid sequence in the mixture of unknown concentration.

It has been found that sensitivity to a range of target nucleic acid concentration of at least six orders of magnitude is possible because the amplifications are monitored in real-time. In addition, sensitivity to as few as 100 ssDNA templates in the background of 40,000 cell-equivalents of complex genomic DNA can be obtained.

The invention further advantageously provides a way to process the fluorescence data to reliably analyze the effect of different reaction conditions on the amplification kinetics. Since multiple amplifications can be monitored simultaneously, the effect of many different reaction variables can be assessed rapidly. This is useful in optimizing amplification reactions for optimum yield and efficiency.

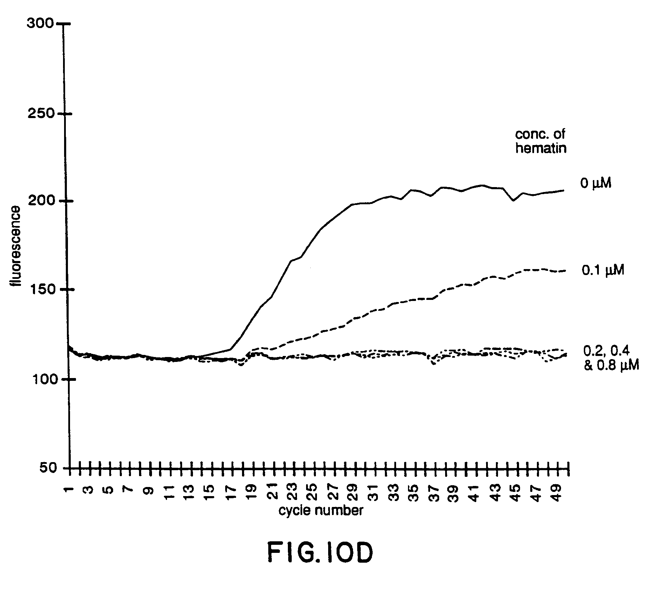

Real-time monitoring also gives the advantage of detecting instances of partial inhibition of PCR, which can greatly affect the ability to quantitate accurately. As shown in Fig. 10D, these instances can be detected by their reaction profile so that these samples can be repurified and tested again. In addition, instances of total inhibition, which might otherwise lead to the false conclusion that there is no nucleic acid target in a sample, can be detected in that there is the expectation that given enough cycles, even PCRs without target DNA will produce fluorescence that is due to nonspecific amplification products. If such fluorescence is not seen by the expected cycle, the presence of inhibitors can be inferred.

A further advantage of the present invention is that the need for additional time-consuming manipulations to determine the yield of many reactions at the end of the amplification is eliminated. Thus, the need for downstream hybridization or gel electrophoresis, for example, is avoided.

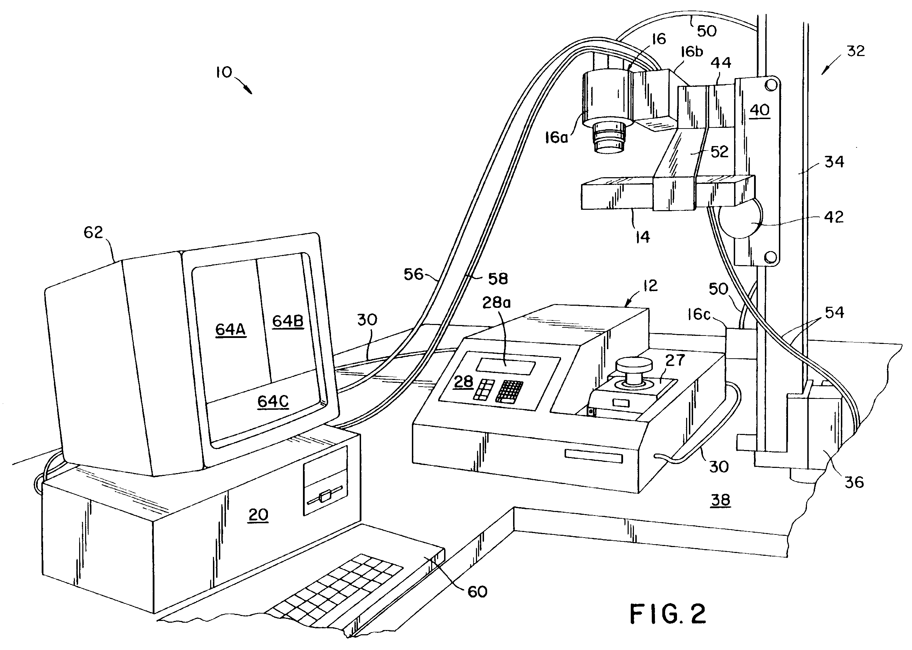

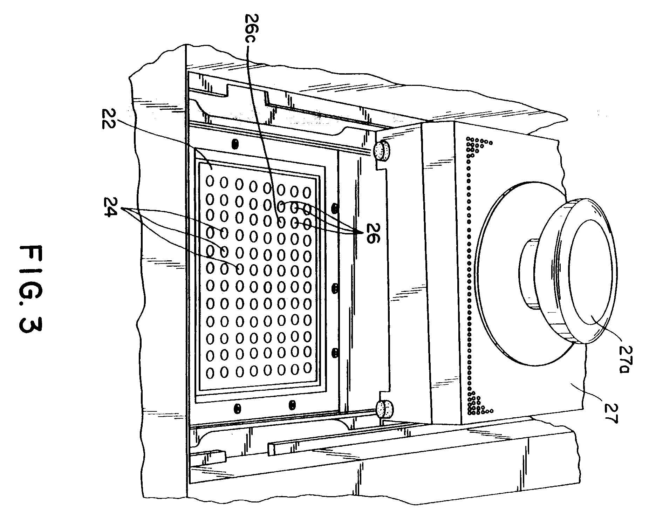

Although the non-fiber-optic embodiment of the present invention illustrated in Figs. 2 and 3 has numerous advantages as described above, the sensor, which preferably is a video camera, is positioned a significant distance from the reaction mixtures in the heat-conducting member to minimize parallax. Accordingly, the light source also is significantly spaced from the reaction mixtures in the heat-conducting member in order to provide uniform illumination and avoid interference with the sensor's vision. This arrangement generally requires a significant amount of space which, in turn, may require an entire darkroom to be allocated to the apparatus. However, many clinical laboratories do not have darkrooms or additional space to allocate to constructing a darkroom. Although X-ray rooms, which can readily be found in clinical laboratories, can provide a suitable environment for the optical paths of the excitation light and fluorescence emissions, the space in these rooms is essentially completely allocated to X-ray equipment.

According to a further embodiment of the present invention, an excitation light and fluorescence sensing configuration is incorporated into the apparatus so that the optical paths of the excitation light and fluorescence emissions can be folded to make the apparatus more compact and to simplify enclosing the optical paths in a light-tight environment. In this manner, the apparatus can be readily used on the bench without the need of a darkroom.

According to this embodiment, the apparatus for monitoring multiple nucleic acid amplifications simultaneously includes a thermal cycler having a heat-conducting member that includes multiple recesses formed therein for receiving nucleic acid amplification mixtures. A housing is positioned over the heat-conducting member so that a light-tight chamber is formed. A light source is arranged to emit light in the housing chamber to excite amplification mixtures disposed therein. A dichroic mirror is positioned in the housing and above the recesses. The dichroic mirror is transmissive to light having a first wavelength and reflective to light having a second wavelength that differs from the first wavelength. In the preferred embodiment, the mirror is a low pass dichroic mirror. The mirror is constructed such that it is transparent or transmissive to light having a wavelength corresponding to the wavelength of light generated by the light source and reflective to light having a wavelength corresponding to the wavelength of fluorescence emitted by the amplification mixtures when exposed to the excitation light. Alternatively, a high pass dichroic mirror can be used. In that case, the mirror is reflective to light corresponding to the wavelength of light generated by the light source and transparent or transmissive to light having a wavelength corresponding to the wavelength of fluorescence emitted by the amplification mixtures when exposed to the excitation light. A sensor also is arranged in the housing for sensing fluorescence emitted from the amplification mixtures. In the low pass dichroic mirror arrangement, the sensor is arranged to receive fluorescence reflected from the mirror. However, in the high pass arrangement, the sensor and light source positions are reversed.

With this arrangement, the excitation light source and sensor both can be positioned relatively close to the heat-conducting member, e.g., from about 6 to 12 inches therefrom, thereby permitting the optical paths of the excitation light source and the fluorescence emissions to take up a relatively small amount of space. The compactness of this arrangement facilitates enclosing the optical paths in the housing, which is coupled to the thermal cycler to effectively form a light-tight chamber or darkroom for the excitation and amplification mixture emission light. In this manner, the apparatus can be used most anywhere, without the need for dedicating a darkroom to the apparatus. The housing further advantageously prevents extraneous light, such as light emitted from monitors used with the apparatus and LED's associated with the thermal cycler and computer equipment, from reaching the sensor.

A further advantage of having the sensor positioned very close to the heat conducting member in which the nucleic acid amplification mixtures are to be disposed is that sensitivity requirements of the sensor can be reduced. Since the sensor is moved closer to the heat-conducting member, the intensity of the light that is emitted from the reaction mixtures and reaches the sensor increases. Accordingly, when using a CCD camera type sensor, as in the preferred embodiment, a less expensive CCD camera with acceptable low light response and low thermal noise can be substituted for a relatively expensive, cooled CCD camera which is generally required when sensitivity requirements are greater.

The close proximity of the light source to the reaction mixture recesses also provides advantages. By moving the light source closer to the reaction mixture recesses, the intensity of the excitation light, which reaches the reaction mixtures that are placed in the recesses, increases, thereby increasing the intensity of the fluorescence emissions. In this manner, smaller concentrations of the target sequence can be detected at an earlier stage of thermal cycling. In addition, some of the increased fluorescence can be traded for better wavelength resolution by using a filter with a more narrow band pass. In this manner, a broader range of wavelengths can be detected and labels with very close wavelengths can be distinguished.

According to another aspect of this embodiment, a shutter is coupled to the light source to intermittently expose the reaction mixtures to the excitation light. Preferably, the shutter is timed to expose the mixtures to the excitation light during the annealing/extension phase where maximum fluorescence generally can be exhibited. This configuration reduces the mixture or sample exposure to the excitation light, which can be very intense due to its close proximity to the heat-conducting member. By minimizing sample exposure to the excitation light, which typically is UV light, but can be of other wavelengths, the shutter improves the system performance by increasing sample stability. Otherwise the intense excitation light can cause photo-deactivation of the sample which is commonly known as "bleaching".

According to a further aspect of this embodiment, a field lens is positioned between the dichroic mirror and the sensor to minimize parallax across the field therebetween.

A further advantageous feature of the invention involves a heated window which is provided immediately above the heat-conducting member and can actually be placed on the tubes containing the amplification mixtures. The heated window prevents heat loss from the reaction tubes, helps maintain the desired thermal cycling temperature profiles of the mixtures and minimizes reflux, while permitting the transmission of light therethrough. According to the preferred embodiment, the heated window is maintained at the denaturation temperature which is generally about 95-105°C.

A filter or wheel of filters preferably is coupled to the sensor so that the sensor can selectively detect light of different wavelengths. The filter(s) also can prevent excitation light from reaching the sensor. By using a plurality of filters, which can be arranged on a filter wheel, the filter can be readily changed during a particular annealing/extension phase, for example, so that emissions of different wavelengths, for example, from different homogeneous nuclease probes (described below), can be monitored. Different emission wavelengths and, thus, nucleic acid sequences in a given sample can be monitored and the presence of the target sequence determined.

In addition, the high sensitivity of the system permits very accurate detection of fluorescence that is produced using a homogenous assay system such as that described in U.S. Patent No. 5,210,015 to Gelfand et al. This assay system uses the 5' to 3' nuclease activity of a nucleic acid polymerase to cleave annealed, labeled oligonucleotides from hybridized probe target duplexes and release labeled oligonucleotide fragments for detection. This assay is particularly useful for detecting multiple nucleic acid targets in the same amplification reaction mixture. Such probes also are useful in confirming target nucleic acid presence when there are nonspeciflc amplification products.

The homogenous assay uses such a probe whose fluorescence is normally quenched by fluorescence energy transfer, or FET, to a second label. The 5' nuclease activity of the DNA polymerase separates the fluorophore from the probe and quencher, disrupting the FET and restoring the fluorescence. No processing is required to detect this change, which may be seen using the monitoring apparatus of the present invention that incorporates the dichroic mirror arrangement. These probes, if labeled with different fluorophores, can be used to detect multiple nucleic acid targets.

The following terms are used in this specification. The accompanying definitions are provided to aid disclosure, rather than limit the invention.

The term "target nucleic acid sequence" refers to a purified or partially purified nucleic acid to be amplified.

The term "amplification reaction mixture" refers to an aqueous solution comprising the various reagents used to amplify a target nucleic acid. These include enzymes, aqueous buffers, salts, amplification primers, target nucleic acid, and nucleoside triphosphates. Depending upon the context, the mixture can be either a complete or an incomplete amplification reaction mixture.

The term "primer" refers to an oligonucleotide capable of acting as a point of initiation of DNA synthesis when annealed to a nucleic acid template under conditions in which synthesis of a primer extension product is initiated, i.e., in the presence of four different nucleotide triphosphates and a DNA polymerase in an appropriate buffer (pH, ionic strength, cofactors, etc.) and at a suitable temperature.

The term "template" refers to a portion of the target nucleic sequence to which the primer anneals.

The above is a brief description of some deficiencies in the prior art and advantages of the present invention.

Other features, advantages and embodiments of the invention will be apparent to those skilled in the art from the following description, accompanying drawings and appended claims.
Fig. 1 graphically shows continuous, real-time monitoring of a PCR;
Fig. 1A is an enlarged view of the area within line 1A in Fig. 1;
Fig. 2 is a perspective view of the amplification apparatus in accordance with the principles of the present invention;
Fig. 3 is an enlarged section of the thermal cycler of Fig. 1 to show the thermal cycler heat exchanger block and cover therefor;
Fig. 4 is a block diagram of the apparatus shown in Fig. 2;
Fig. 5 shows examples of digitized images of multiple reaction mixtures;
Fig. 6 illustrates a selected pixel array for averaging to obtain a single fluorescence value;
Fig. 7 represents cycle-to-cycle fluorescence measurement drift;
Fig. 8A shows multiple fluorescence profiles;
Fig. 8B shows the fluorescence values of Fig. 8A after normalization;
Fig. 8C shows gel electrophoresis of the amplification products represented by the profiles in Figs. 8A and B;
Fig. 9 shows the linear relationship between the log of starting template copies and the number of cycles required to reach a selected fluorescence value;
Figs. 10A-D represent the effect of changes in reaction conditions;
Figs. 11-13 are simplified flow charts of the steps for obtaining the fluorescence values;
Fig. 14 is a perspective view of the amplification apparatus of the present invention illustrating a further embodiment of the excitation light and emissions/fluorescence detector arrangement in partial section; and
Fig. 15 is an enlarged view of the excitation light and emissions/fluorescence detector arrangement of Fig. 14 in partial section;
Fig. 16 shows a transmissivity v. wavelength curve that is representative of a dichroic mirror constructed in accordance with principles of the present invention; and
Fig. 17 is a top view of a heating window for the upper portion of the reaction mixture containing vessels according to the present invention.

The present invention involves nucleic acid amplification and the detection, monitoring and quantitation of amplification products. In order to facilitate understanding of the amplification data collection and processing system of the present invention, a summary of nucleic acid amplification processes especially suited for use in conjunction with the invention will first be discussed.

Those of skill will recognize that the present invention requires amplification of the duplex form of nucleic acid. There exist well-known methods for amplifying nucleic acids. The means for amplification are not critical and this invention will work with any method where nucleic duplexes are generated. The various methods are reviewed in Bio/Technology 8:290-293, April 1990. They include, but are not limited to PCR, LCR, Qβ and 3SR. Although 3SR and Qβ do not involve thermal cycling, the result of their amplifications can be monitored by the fluorescence detecting arrangement discussed below and analyzed in accordance with the principles of the present invention. Each method is briefly described below.

PCR amplification of DNA involves repeated cycles of heat denaturing the DNA, annealing two oligonucleotide primers to sequences that flank the DNA segment to be amplified, and extending the annealed primers with DNA polymerase. The primers hybridize to opposite strands of the target sequence and are oriented so that DNA synthesis by the polymerase proceeds across the regions between the primers, each successive cycle essentially doubling the amount of DNA synthesized in the previous cycle. This results in the exponential accumulation of the specific target fragment, at a rate of approximately 2n per cycle, where n is the number of cycles. A complete review of this technology can be found in PCR Technology: Principles and Applications, Ed. Erlich H.A., Stockton Press, New York 1989.

Taq DNA polymerase is preferred when PCR is used in conjunction with the present invention although this is not an essential aspect of the invention. Taq polymerase, a thermostable polymerase, is active at high temperatures. Methods for the preparation of Taq are disclosed in U.S. Patent No. 4,889,818 and incorporated by reference. However, other thermostable DNA polymerases isolated from other Thermus species or non-Thermus species (e.g., Thermus thermophilus or Thermotoga maritima), as well as non-thermostable DNA polymerase such as T4 DNA polymerase, T7 DNA polymerase, E. coli DNA polymerase I, or the Klenow fragment of E. coli, can also be used in PCR. Methods for providing thermostable DNA polymerases are provided in International Patent Applications with publication Nos.WO-A- 91/09950 and
WO-A- 92/03556

The ligase chain reaction is described in International Patent Application with publication No. WO 89/09835, The process involves the use of ligase to join oligonucleotide segments that anneal to the target nucleic acid. Ligase chain reaction (LCR) results in amplification of an original target molecule and can provide millions of copies of product DNA. Consequently, the LCR results in a net increase in double-stranded DNA. The present detection methods are applicable to LCR, as well as PCR. LCR typically requires some means for detecting the product DNA such as an oligonucleotide probe. When used in conjunction with the disclosed methods for detecting amplification products, such means are unnecessary, and the LCR result is immediately detectable.

Another amplification scheme, Q-beta replicase, exploits the use of the replicase from the RNA bacteriophage Qβ. In this amplification scheme, a modified recombinant bacteriophage genome with a sequence specific for the targeted sequence is initially ligated to the nucleic acid to be tested. Following enrichment of the duplexes formed between the bacteriophage probe and the nucleic acid in a sample, Qβ replicase is added, which, upon recognizing the retained recombinant genome, begins making a large number of copies.

The Qβ system does not require primer sequences and there is no heat denaturation step as with the PCR and LCR amplification systems. The reaction occurs at one temperature, typically 37°C. The preferred template is a substrate for the Qβ replicase, midvariant-1 RNA. A very large increase in the templates is achieved through the use of this system. A review of this amplification system can be found in the International Patent Application Pub. No. WO 87/06270 and in Lizardi et al., 1988, Bio/Technology 6:1197-1202.

The 3SR system is a variation of an in vitro transcription-based amplification system. A transcription-based amplification system (TAS) involves the use of primers that encode a promoter sequence as well as a complementary sequence to the target strand to generate DNA copies of a target strand and the production of RNA copies from the DNA copies with an RNA polymerase. See, e.g., Example 9B of U.S. Patent No. 4,683,202 and European Patent Application with publication No.
EP-A-0 310,229. The 3SR System is a system which uses three enzymes to carry out an isothermal replication of target nucleic acids.

The system begins with a target of single-stranded RNA to which a T7 RNA DNA primer is bound. By extension of the primer with reverse transcriptase, a cDNA is formed, and RNAseH treatment frees the cDNA from the heteroduplex. A second primer is bound to the cDNA and a double-stranded cDNA is formed by reverse transcriptase treatment. One (or both) of the primers encodes a promoter, e.g., the promoter for T7 RNA polymerase, so that the double-stranded cDNA is a transcription template for RNA polymerase.

Transcription competent cDNAs yield antisense RNA copies of the original target. The transcripts are then converted by the reverse transcriptase to double-stranded cDNA containing double-stranded promoters, optionally on both ends in an inverted repeat orientation. These DNAs can yield RNAs, which can reenter the cycle. A more complete description of the 3SR system can be found in Guatelli et al., 1990, Proc. Natl. Acad. Sci. USA 87:1874-1878, and European Patent Application with publication No. EP-A-0 329 822. The TAS system is also described in Gingeras et al., in Innis et al. eds, 1990, PCR Protocols, Academic Press, San Diego,

According to the present invention, nucleic acid amplification is monitored by detecting fluorescence emitted when fluorescent dye such as an intercalating fluorescent dye, provided in the reaction mixture, binds with the double-stranded nucleic acid during each annealing/extension phase as the mixture is cycled between two temperatures (thermal cycling).

An increase in fluorescence indicates a positive amplification of target nucleic acid. Suitable intercalating agents or dyes include, but are not limited to ethidium bromide, propidium bromide, proflavine, acridine orange, acriflavine, fluorcoumanin, ellipticine, daunomycin, chloroquine, distamycin D, chromomycin, homidium, mithramycin, ruthenium polypyridyls, anthramycin, methidium bromide, 2-[2-(4-hydroxyphenyl)-6-benzimidazole-6-(1-methyl-4-piperazye) benzimidazole trihydrochloride and the like.

Fluorophores and DNA binding chromophores described in the art are suitable for use in the 5' to 3' nuclease assay disclosed in U.S. Patent No. 5,210,015 are also useful in the present invention. Suitable donor fluorophores and quenchers are chosen such that the emission spectrum of the donor fluorophore overlaps with the absorption spectrum of the quencher. Ideally, the fluorophores should have a high Stokes shift (a large difference between the wavelength for maximum absorption and the wavelength for maximum emission) to minimize interference by scattered excitation light.

Suitable labels which are well known in the art include, but are not limited to, fluoroscein and derivatives such as FAM, HEX, TET, and JOE; rhodamine and derivatives such as Texas Red, ROX, and TAMRA; Lucifer Yellow, and coumarin derivatives such as 7-Me₂N-coumarin-4-acetate, 7-OH-4-CH₃-coumarin-3-acetate, and 7-NH₂-4-CH₃-coumarin-3-acetate (AMCA). FAM, HEX, TET, JOE, ROX, and TAMRA are marketed by Perkin Elmer, Applied Biosystems Division (Foster City, CA). Texas Red and many other suitable compounds are marketed by Molecular Probes (Eugene, OR). Examples of chemiluminescent and bioluminescent compounds that may be suitable for use as the energy donor include luminol (aminophthalhydrazide) and derivatives, and Luciferases.

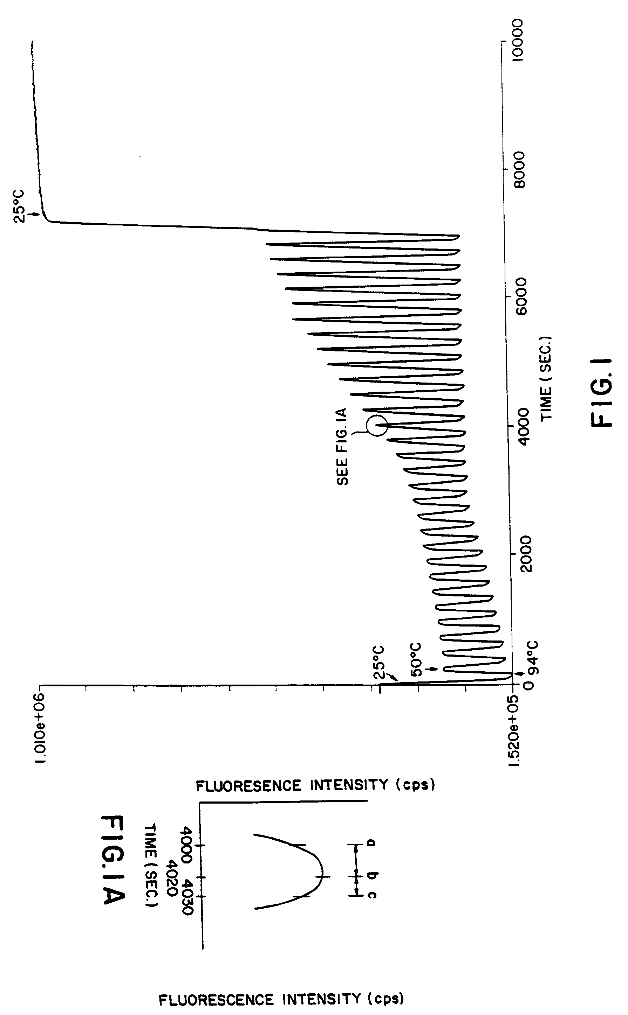

Referring to Fig. 1, a fluorescence trace indicative of DNA amplification for a single polymerase chain reaction (PCR), which involves cycling the temperature of the reaction mixture between two temperatures, e.g., 94°C and 50°C, is shown. The reaction mixture included an intercalating fluorescent DNA binding dye, ethidium bromide (EtBr), which intercalates, or binds, the double-stranded PCR products and fluoresces during each annealing/extension phase of the reaction. Consequently, the resulting fluorescence increases as the amount of double-stranded DNA increases. As illustrated in Fig. 1, fluorescence intensity rises and falls inversely with temperature. The fluorescence intensity is minimum at the denaturation temperature (94°C) and maximum at the annealing/extension temperature (50°C). Thus, the fluorescence intensity at 50°C shows a cycle-dependent increase reflecting an increase in the amount of double-stranded DNA. When the thermal cycler returned to 25°C, after the 30 cycles were completed, the fluorescence increased to a final value greater than three times the initial fluorescence value at 25°C. On the other hand, the fluorescence minima at the denaturation temperature do not significantly increase, presumably because at this temperature there is no dsDNA for EtBr to bind.

As disclosed in U.S. Patent Application Serial No. 07/695,201, this data was collected using a fiber-optic lead and a spectra-fluorometer. The fiber-optic lead was used to input excitation light directly to the tube containing the reaction mixture and the tube positioned in a heating/cooling block of a thermal cycler. The same fiber-optic lead was used to return fluorescent emissions back to the spectra-fluorometer, where the value corresponding to emitted fluorescence was read. With this arrangement, the generation of amplification products can be monitored while the reaction is in progress as evidenced by the data in Fig. 1. Since the signal generated by the binding agent can be detected without having to open the reaction tube, the signal can be monitored throughout before, during, and after amplification process.

The apparatus for detecting a single PCR and providing the data in Fig. 1 was set up as follows: a Spex-Fluorolog-2 fluorometer with a fiber-optic accessory (Spex Catalog No. 1950) was set to emit excitation light at 500 nm with a bandwidth of approximately 3.4 nm. A GG 435 nm cut off filter was used to exclude second order light (Purchased from Melles Grist Inc.). The emission light was detected at 570 nm with a bandwidth of approximately 13.6 nm. A OG530 filter (530 nm cut off) was used to remove excitation light.

The reaction tube, a 0.5 ml polypropylene tube, contained 60 ng human male DNA. The top of the tube was cut away for attaching the fiber-optic cable. The fiber-optic cable was glued to the top of the reaction tube with epoxy. The emission light was collected through the oil overlay in the tube. A black "shroud" was built around the tube and the reaction was placed in the thermal cycler. The thermal cycler was programmed to cycle between 94°C and 50°C for 1 minute each, for 30 cycles, followed by continuous incubation at 25°C. The fluorometer and thermal cycler were started simultaneously. The parameters of the fluorometer were: time-based scan with 5 second integration time; the emission signal was ratioed to that of the excitation light to control for changes in source intensity.

In accordance with the present invention, an apparatus is provided for real-time detection of the amplification of multiple amplification reaction mixtures, simultaneously. Each reaction mixture contains a target nucleic acid sequence. Thus, using a thermal cycler (e.g., a thermal cycler commercially available from Perkin Elmer Cetus Instruments) having a heating and cooling block capable of holding up to 48 reaction tubes, for example, 48 amplification reactions can be carried out and PCR amplification product in all 48 samples detected simultaneously without handling the samples, opening tubes, or interrupting the cycling reaction. The number of reactions monitored simultaneously is limited only by the capacity of the heating and cooling block. Thus, with a 96 well heating and cooling block, 96 amplification reactions can be monitored and the amplification product detected simultaneously. As in the example case described above, it should be understood that although the embodiments discussed below are described in conjunction with PCRs and signals generated by a specific fluorescent binding agent indicative of amplification of a target DNA sequence in certain examples, these embodiments can be used with other amplification processes or binding agents such as those summarized above and disclosed in U.S. Patent No. 5,210,015 to Gelfand et al.

In a first embodiment of the multiple amplification reaction mixture detection apparatus, a suitable optical system moves the excitation light from a source to multiple reaction tubes that are positioned in a thermal cycler and measures the emission light from each tube. Such an optical system can comprise multiple fiber-optic leads that simultaneously read all of the PCR tubes undergoing thermal cycling. Only a single fluorometer is needed to read fluorescence from the reaction tubes, as each fiber-optic can be read rapidly one at a time, for example, during the time frame of a PCR temperature soak (e.g., an annealing/extension interval such as the interval between a-c illustrated in Fig. 1A). It will be apparent to one skilled in the art that such a detection system is not necessarily limited to a particular thermal cycler machine or reaction vessel.

The amplification detection arrangements incorporate fiber-optic leads to transmit excitation light and fluorescence emissions. So long as the reaction wells, or tubes, are light-sealed to prevent external light sources from influencing fluorescence detection, any over plate, tube cap, or lid apparatus that comprises or can be attached to a fiber-optic lead is suitable. In one embodiment of the invention, the reaction tube lids can be removed to accommodate the fiber-optic. However, use of a reaction vessel that has a clear or translucent cap eliminates the need to insert the cable into the tube. It will be apparent that reaction tubes capable of accommodating a fiber-optic cable, without the cable physically contacting the amplification reaction components, are desirable.

Referring to Fig. 2, a further embodiment of an apparatus for real-time detection of the amplification of multiple target nucleic acid sequences, simultaneously, is shown and designated with reference numeral 10. This embodiment advantageously eliminates the need for the light input and fluorescence output fiber-optic leads described above and the accompanying problems associated with coupling the fiber-optics to the reaction tubes. As discussed above, apparatus 10 will be described in conjunction with PCR amplification of a DNA target sequence using a fluorescent binding agent signal generator for purposes of simplification, but is not intended to be limited to use with such an amplification process, nucleic acid or amplification signal generator.

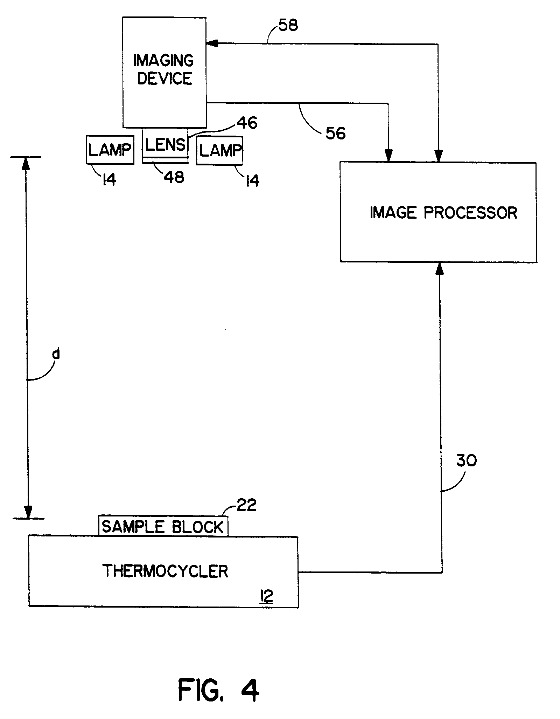

Apparatus 10 generally comprises a thermal cycler 12 for heating and cooling reaction chamber tubes or vessels 26 (preloaded with the nucleic acid(s) to be amplified), laterally spaced excitation light sources or lamps 14, imaging device 16 (comprising CCD-camera 16a, camera controller 16b and cooler 16c), and image processor 20, which can be a PC with a frame grabber card such as the PC VISION PLUS commercially available from ITEX Inc. and an IEEE 488 interface card. These elements are shown in block diagram in Fig. 4. Although thermal cycler 12 is conventional in construction according to the preferred embodiment, a summary of certain features of thermal cycler 12 is provided below to aid in understanding the disclosure of the present invention.

Thermal cycler 12 includes a heat exchanger 22 for heating and cooling reaction tubes 26. Heat exchanger 22 is a heat-conducting block, preferably aluminum, having a plurality of recesses 24 formed therein and sized to allow a given number of reaction tubes 26 (e.g., 0.5 ml Eppendorf tubes) to fit therein. The purpose of heat exchanger 22 is to support reaction tubes 26 and to act as a heat exchanger to transfer thermal energy to and from the fluids stored in the tubes such that the reaction components may be incubated at various temperatures for user-defined times. Thus, thermal cycler 12 also includes a system for heating and cooling the heat exchanger and means, such as a computer system, for controlling the heating and cooling system to heat and cool the heat exchanger and reaction tubes as is conventional in the art. The user programs the computer or means for controlling the heating and cooling of heat exchanger and reaction tubes to a desired temperature profile, such as that illustrated in Fig. 1, through display/keyboard user interface 28.

The heat exchanger (heat conducting block), means for heating and cooling the heat exchanger and means for controlling the heating and cooling of the heat exchanger as well as the entire thermal cycler, can be constructed in accordance with U.S. Patent No. 5,038,852. Suitable thermal cyclers are commercially available such as the GeneAmp PCR system 9600 thermal cycler (Perkin-Elmer, Norwalk, CT) depicted in Fig. 2.

It should be understood, however, that other devices for heating and cooling the preloaded reaction tubes can be used without departing from the scope of the invention. It is only necessary that whatever device is used for heating and cooling the reaction tubes, be capable of reaching and sustaining the temperatures involved and achieve the desired temperature versus time profile. Thus, for purposes of nucleic acid amplification, such a device should be capable of cycling the temperature of the amplification reaction mixture between a denaturing temperature T₁ (which can be in the range of about 80-105°C and preferably 90-100°C) and an annealing/extension temperature T₂ (which can be in the range of about 30-90°C and preferably 50-70°C) where T₁ > T₂ as is known to those skilled in the art.

Cover block 27, including handle 27a, is slidably coupled to the heat exchanger for covering or uncovering heat exchanger 22 as is conventional in the art. Cover block 27 is maintained in the latter position as shown in Fig. 3 during the amplification/detection process of the present invention embodied in Fig. 2. This permits camera 16a to receive the fluorescence signals emitted from within the amplification reaction tubes.

Thermal cycler 12 also is provided with a means for sending output data indicative of the heat exchanger or amplification reaction mixture temperature profile, such as cycle number, temperature, and time, to image processor 20 over line 30. An RS232 bus for transmitting such data is provided with a GeneAmp PCR system 9600 thermal cycler, for example. LED display panel 28a also displays such output data to the user during operation.

CCD-camera 16a is positioned above heat exchanger 22 and the reaction tubes to collect fluorescence signals emitted from the reaction mixtures through the upper ends of the tubes as the temperature of the mixtures is cycled by device 12 during amplification. It is important that camera 16a is positioned as far as is practical from heat exchanger 22 in order to reduce parallax effects on the fluorescence image captured by camera 16a. However, camera 16a must be sufficiently close to the heat exchanger and reaction tubes to minimize background noise from other light sources and so that sufficient light from reaction tubes 26 can be captured by the camera. It has been found that an optimum distance d, between the heat exchanger 22 and camera lens 46, which is directed toward the heat exchanger, is in the range of about 6 inches to 5 feet depending on the lens used. For example, d preferably is about 2 feet when a 70 mm lens is used. A band pass interference filter 48, preferably a 600 nm filter, is placed in front of the lens to limit detection to the desired wavelength, since the fluorescence of interest has wavelengths of about 600 nm.

Referring to Fig. 2, camera 16a is shown mounted to a conventional photographic copy stand 32 for positioning the camera above heat exchanger 22 and reaction tubes 26. Copy stand 32 includes a vertical support member 34 which is supported in bracket 36. Bracket 36 is secured to mounting surface 38 which supports apparatus 10. Slide member 40 is slidably mounted to vertical member 34 for movement along the vertical and is provided with knob 42 which upon turning cooperates with slide member 40 to lock slide member 40 in a feed position as is conventional to one of ordinary skill. Camera bracket 44 has one end secured to slide member 40 and another end secured to camera 16a. Thus, the height of camera 16a can be adjusted through slide member 40 to the desired height above the heat exchanger and reaction tubes. Camera 16a preferably is a conventional computer-controlled, cooled CCD-camera. Cooling fluid is circulated through camera 16a through multiconduit line 50. The camera is preferably cooled in order to provide higher quality images with less noise. One suitable camera is a CCD-camera commercially available from Photometrics (Tucson, AZ) under the name STAR 1. It uses a thermoelectrically cooled CCD array with a 12-bit per pixel resolution. It also permits exposure times to be varied to change sensitivity to the fluorescence being detected. An IEEE 488 bus (designated with a reference numeral 58) between camera 16a and image processor 20 is provided so that image processor 20 can control the exposure of an image of the reaction tubes and the time period in which the image is taken.

Referring to Fig. 4, two UV lamps 14 are laterally spaced about the optical path between camera 16a and heat exchanger 22. It is only important in this regard that lamps 14 are sufficiently close to camera lens 46 so that their housings do not interfere with the optical path between all of the reaction tubes and camera lens 46. To this end, lamps 14 can be suspended from camera bracket 44 via bracket 52. Lamps 14 also are parallel to the heat exchanger so that excitation light is provided to the reaction tubes in a uniform manner. Lines 54 extend from lamps 14 and are adapted to fit in a standard 115 volt wall socket for providing the desired power to the lamps. The wavelength of UV lamps 14 is selected according to the requirements of the nucleic acid fluorescent binding agent. For example, 302 nm is a desirable wavelength for excitation of ethidium bromide. A suitable 302 nm mid-range UV lamp is commercially available from U.V. Products, Inc. (San Gabriel, CA) under the name Chroma-Vue.

The image taken by camera 16a is transmitted to image processor 20 via line 56 which, when using the STAR 1 system as discussed above, is an RS170 line. Image processor 20 includes a frame grabber for forming digital images from camera data on line 56. Although camera 16a includes a 12-bit digital output, the analog output of the camera is used where image processor 20 is an 8-bit image processor. In such a case, the image processor 20 includes the frame grabber to create 8-bit digital images from the analog signal on line 56 so that image processor 20 can manipulate the collected images as digital data.

Thermal cycler output data line 30 also is coupled to image processor 20 for providing the processor with the temperature profile, time, and cycle of the amplification in progress. In this manner, processor 20 can be programmed to send a trigger signal via line 58 to camera controller 18, which controls the opening of the shutter for camera 16a, at preselected times during the course of amplification. Image processor 20 includes a IEEE 488 bus card for communicating with IEEE 488 line 58, to control camera 16a. Processor 20 also is provided with a conventional keyboard and/or mouse user interface 60/64 and monitor 62. A general overview of the operation of apparatus 10 is provided below.

Multiple reaction tubes 26 each containing a reaction mixture (e.g., nucleic acid(s) to be amplified, a thermostable enzyme to catalyze polymerization, specific oligonucleotide primers, and four different nucleotide triphosphates) are placed in heat exchanger block 22 (Fig. 3). The reaction tubes can be sealed with conventional caps, a vapor barrier mineral oil or Ampliwax™ brand sealant. The thermal cycler is activated and the excitation lamps, which are directed toward the reaction tubes, are energized. Responsive to a signal from camera controller 16b, camera 16a captures an image of all of the reaction tubes 26 which image reflects the various levels of fluorescence at the first annealing/extension phase of a cycle. This analog image is sent to the image processor where the frame grabber digitizes the image since it has an analog/digital converter.

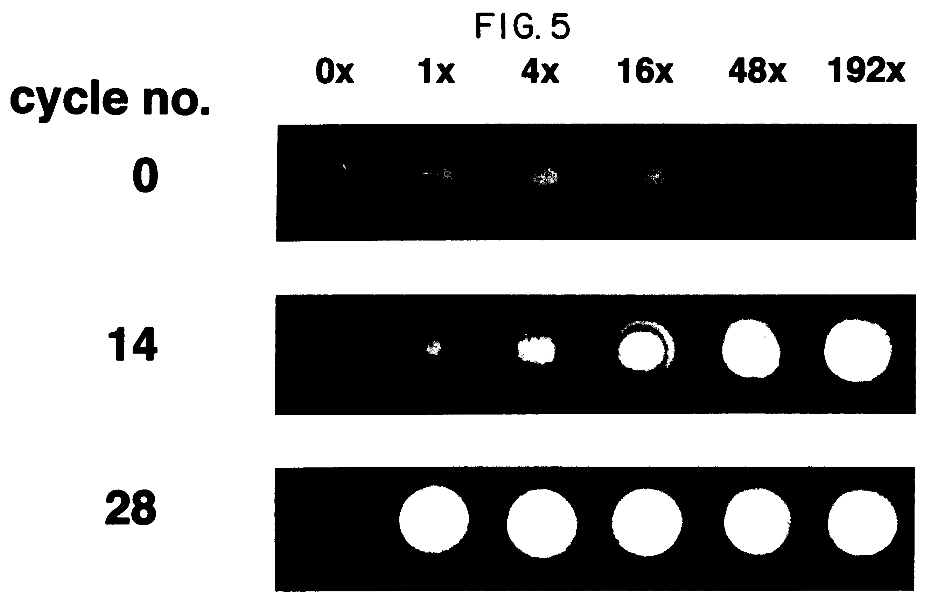

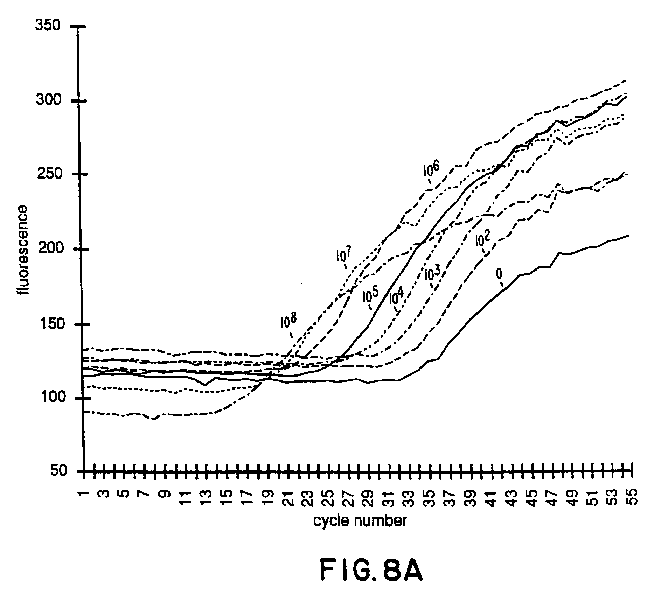

Fig. 5 shows portions of three such digitized images taken during the course of six simultaneous amplifications. The processor averages the intensity of light emitted from each reaction tube during the noted annealing/extension phase and normalizes those intensity values to determine a single fluorescence value for each reaction tube. The normalization process is discussed below in more detail in connection with Figs. 8A-C. This is repeated for each cycle of the amplification process. The normalized fluorescence values are saved to a computer spreadsheet for subsequent analysis and graphing. These normalized fluorescence values are further processed to determine the initial amount of the target nucleic acid sequence (i.e., the starting number of target nucleic acid templates before amplification) or to analyze the effect of amplification reaction conditions on the kinetics of the reaction. A more detailed discussion of the fluorescence value acquisition and processing steps, summarized above, is provided below.

The timing of each camera exposure to the emitted fluorescence first will be discussed. Since the maximum fluorescence occurs at the annealing/extension phase of each cycle in an amplification process undergoing thermal cycling (e.g., see interval a-c Fig. 1A) and this is the indication of the level of nucleic acid amplification, when only one image is taken in a given cycle by camera 16a it should be taken in this phase of the cycle because that is when you get the strongest signal and signals during this interval best represent the amount of material being made. However, as other data throughout the reaction can be useful in analyzing the entire course of the amplification, it should be understood that it may be desirable to collect more than one image per cycle. For example, each image can be taken 20 seconds after the annealing/extension temperature is reached as indicated by reference character b in Fig. 1A. In this example case, when the annealing temperature is 68°C, processor 20 sends a signal, 20 seconds after the 68°C temperature is indicated by the thermal cycler 12 over RS232 line 30, to camera controller 16a to trigger the camera shutter (not shown). The exposure time can vary from application to application as would be apparent to one of ordinary skill. The triggering event to open the camera shutter obviously can be done manually with the operator monitoring the temperature and time output data displayed on display 28a.

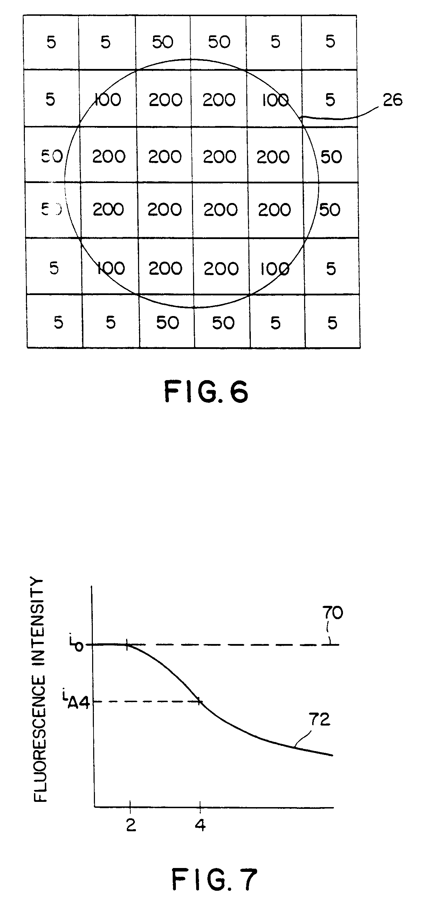

The CCD-camera sums up the light received during the exposure and produces an image indicative of the fluorescence of all of the reaction tubes in the heat exchanger at that time interval. This image is sent over line 56 to image processor 20. Monitor 62, which is coupled to processor 20, can display three windows labeled 64A-C in Fig. 5. Window 64A can monitor the camera's view and provide a menu during camera operation for functions such as camera exposure control. Window 64B displays the digital image and facilitates the manual selection of the portion of the image to be processed (e.g., averaged as discussed below). Window 64C displays the command line of the computer operating system. The processor digitizes the image and displays it in window 64B which is used so that the user can assign a group of pixels to an area of the image that encompasses a single image of a reaction tube. This can be accomplished using a mouse to draw boxes around the portions of the digitized image of a reaction tube 26 in window 64B or using a program that looks at the fluorescence of each pixel to determine which pixels belong to which reaction tube as would be apparent to one of ordinary skill in the art of computer graphics. This is illustrated in Fig. 6, where a 6x6 pixel array encompasses the image of a single reaction tube 26. It should be understood that this array size has been selected merely for purposes of example and that other array sizes can be used without departing from the scope of the invention. The pixel values "5" indicate insignificant fluorescence and correspond to the fluorescence emitted by the upper surface of the heat exchanger, while the pixel values "200" indicate the maximum fluorescence detected in the 6x6 pixel array and correspond to the fluorescence emitted from the center of the reaction tube which are imaged onto pixels. The pixel values "100" correspond to the fluorescence emitted by a portion of the heat exchanger and reaction tube. Of course, these specific pixel values do not necessarily represent actual values, but are merely exemplary.

Image processor 20 then averages the pixel values in the array assigned to the area encompassing the reaction tube to obtain a single fluorescence value for that reaction tube for cycle 1 of the nucleic acid amplification process. However, obtaining a single fluorescence value for each reaction tube can be accomplished in other ways such as summing the pixel values. The box outlining the 6x6 array and encompassing the first reaction tube is then moved to encompass the next reaction tube where the pixel value averaging step is repeated and a single fluorescence value is obtained until a fluorescence value has been assigned to each tube for cycle 1. Alternatively, the process of obtaining a fluorescence value for each reaction tube 26 for a given cycle can be done as a parallel process. This process of calculating a fluorescence value for each reaction tube is repeated each cycle until the amplification process is complete.

At the outset, i.e., when heat exchanger block 22 is loaded with reaction tubes 26, a control tube 26C is also loaded into one of the wells 24. The purpose of the control tube is to provide a constant fluorescence source against which cycle-to-cycle measurement variations, due to minor temperature variations or drift in the excitation light intensity, for example, can be detected. In this manner, the fluorescence value obtained for each reaction tube can be corrected to compensate for these variations according to the following. The control tube 26C is pre-loaded with a fluorescence dye only, such as ethidium bromide (EtBr). Since the control tube contains no nucleic acid, there is no amplification of its fluorescence during thermal cycling. Thus, the control tube will emit an essentially constant amount of fluorescence when subjected to excitation light during amplification and serves as a base line throughout the amplification process. This is illustrated in Fig. 7, where the fluorescence intensity of the control tube is plotted over several cycles.

Referring to Fig. 7, iₒ is the initial fluorescence of the control tube and establishes the base line. At cycle 2, the recorded intensity of control tube 26 begins to drop off indicating that drift in fluorescence is occurring. Thus, solid line 72 cycle 4 corresponds to apparent intensity values. For example, at cycle 4 when the camera collects data in the annealing/extension phase of the fourth cycle, the apparent value of the control tube fluorescence is i_{A4}. A correction factor iₒ/i_{A4} is then determined for that cycle. Each of the fluorescence values obtained for each reaction tube is multiplied by the correction factor to provide actual fluorescence values. The foregoing is repeated for each cycle. In summary, the correction factor is generally described as iₒ/i_{An} wherein iₒ is the initial fluorescence value of the control tube and i_{An} is the apparent fluorescence value of the control tube taken during cycle number n. Each fluorescence value obtained for a reaction tube by, for example, averaging (as discussed above) is multiplied by the correction value. The foregoing is repeated for each cycle.

Normalization and quantitation will be described with reference to data generated for a specific example case for exemplary purposes only. That data is illustrated in Fig. 8A. It should be understood that the invention is not intended to be limited to PCR or the particular amplification reaction mixtures, number of amplifications, temperature profile, or interval in which the fluorescence images were taken as will be apparent to one of ordinary skill. It is only necessary to monitor the amplification reaction mixtures differing in starting concentration of a target nucleic acid sequence to quantitate the initial concentration of that target sequence in an amplification reaction mixture where that concentration initially is unknown as will be described in more detail below.

Fig. 8A shows the fluorescence values obtained from eight PCR amplifications monitored simultaneously, in accordance with the principles described above, during thermal cycling for fifty-five cycles with the CCD-camera. Thus, these values represent the values obtained for each reaction tube (e.g., by averaging the respective pixel arrays) corrected for occurrences of measurement variations (e.g., drift) as discussed above. In this example case, seven reaction mixtures contained known concentrations of a specific DNA target sequence (i.e., a known quantity of starting sample) to facilitate quantitation of an unknown starting amount of a sample as will be described in more detail below. The eighth mixture contained no target DNA and served as a control reaction to provide background information. The seven amplifications of known sample were initiated using a dilution series of single-stranded, HIV template DNA.

Prior to making the dilutions, the PCR reaction mixtures were set up in 100 µl volumes containing 10 mM Tris-HCl, pH 8.3; 50 mM KCl; 3 mM MgCl₂; 2.5 U of Taq DNA polymerase (Perkin Elmer, Norwalk, CT); 100 µM each dNTP (Promega Corp., Madison, WI); 4 µg/ml of ethidium bromide (Bio Rad, Richmond, CA); 100 pmole each of HIV specific (gag region) oligonucleotide primers SK145 and SK431 (Perkin Elmer, Norwalk, CT) and 300 ng of human placental DNA (Sigma, St. Louis, MO). An M13 subclone of the HIV *gag* region was used as the target nucleic acid template.

Starting with 10⁸ templates, 10-fold dilutions were made down to 10² templates and used with HIV-specific primers to make a 142 bp PCR product. The fluorescence profiles in Fig. 8A generated from these initial concentrations are designated with reference numerals 10⁸, 10⁷...10², respectively. A control amplification with no added template was also monitored. The fluorescence profile of this mixture, is shown by the line designated with reference numeral 0. To simulate a PCR-based screen of lymphocyte DNA for integrated HIV genomes, all reactions contained 300 ng or 40,000 cell equivalents of human genomic DNA.

Thermal cycling proceeded for 55 cycles in a GeneAmp PCR System 9600 thermal cycler (Perkin-Elmer, Norwalk, CT) with a 2 temperature program: 94°C for 15 sec. denaturing; 68°C for 30 sec. annealing/extension. To provide a "hot-start" as is conventional in the art to improve specificity in the reaction (as described in Chou, Q., Russell, M., Birch D.E., Raymond, J. and Bloch, W. "Prevention of pre-PCR mis-priming and primer dimerization improves low-copy-number simplifications." Nucleic Acids Research 20:1717-1723,1992) an initial hold at 75°C was used during which the MgCl₂ was added (in a 12 µl volume) after the samples had reached this temperature. Fluorescence images for each thermal cycle were taken by the CCD-camera 20 sec. into the 30 sec. hold at the annealing temperature with an exposure of about 1 second. As described above, the heating cover block 27 was not used and was positioned away from the reaction tubes to permit imaging.

As shown in Fig. 8A, the fluorescence from each reaction changes little in early thermal cycles and then rises as detectable amounts of PCR product are generated. The more starting template copies in the reaction, the earlier such a rise in fluorescence occurs. See, for example, the curve designated with the character 10⁸ which designates that that fluorescence curve corresponds to the fluorescence emitted by the reaction mixture starting with the highest concentration of starting templates, i.e., 10⁸ templates. As shown in Fig. 8A, the 10⁸ curve rises first as expected.

However, there is a considerable variance in the initial fluorescence values obtained from the different amplifications even though those values should be the same. They should be the same because the amounts of single-stranded DNA are so small that the varying amounts of it in the reaction tubes have a negligible effect on the total fluorescence expected from a single tube. It is believed that the sources of this variation are inhomogeneity or nonuniformity of illumination, parallax, and variable attenuation of the fluorescence due to the tube caps. Monitoring the amplifications without caps but through a vapor barrier of mineral oil or Ampliwax™ brand sealant reduces, but does not eliminate, this variation.

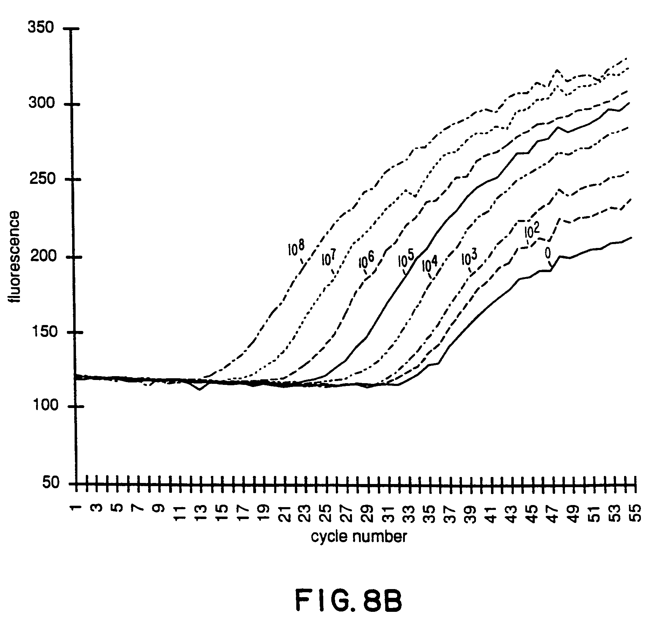

To compensate for inhomogeneity of illumination parallax, and variable attenuation and, thus, the variations shown in Fig. 8A, the processor determines a normalization factor for each amplification. Each factor is determined based on the data collected in cycle 1 and is the ratio of the average initial fluorescence of all reactions to the observed initial fluorescence of each reaction. The processor multiplies all fluorescence values for each amplification by the respective normalization factor. The result of this normalization is shown in Fig. 8B. Thus, for the reaction containing 10⁸ starting template copies, the ratio (normalization factor) is the sum of all eight fluorescence values in cycle 1 divided by 85 which is the initial fluorescence value of reaction 10⁸ as shown in Fig. 8A. The fluorescence values for the reaction marked 10⁸ then are all multiplied by this factor to generate the normalized curve of Fig. 8B. Each reaction curve is similarly normalized. This normalization is based on the assumption that the source of the image variation attenuates or enhances the fluorescence signal proportionately over the entire range of signal intensities.

Referring to Fig. 8B, all reactions essentially have the same initial fluorescence and most of the reaction profiles have a regular spacing consistent with the dilution series used. The profiles are very similar in shape and nearly parallel. The early or logarithmic phase of an efficient PCR involves a doubling of the DNA copy number every cycle. It is predicted that each 10-fold dilution in starting template would require 3.32 additional cycles to bring the yield of PCR product up to a given concentration. Thus, the normalized values look as expected, for much of the range of fluorescence levels. It can be seen by examination of Fig. 8B that there are close to three cycles separating each of the dilutions except for those starting with 10² copies and 0 copies. It is also apparent that this cycle-number-offset is persisting past the logarithmic phase of the PCRs. In contrast, the number of cycles separating the dilutions in the unnormalized data of Fig. 8A vary widely.

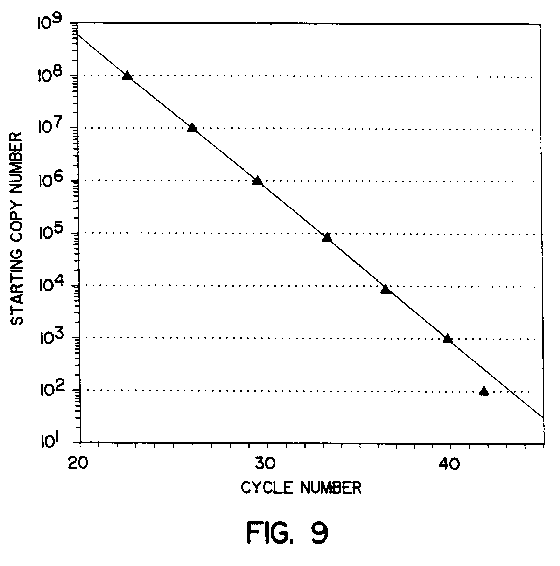

Using the normalized fluorescence data, such as the data shown in Fig. 8B, the initial amount of target nucleic acid can be quantitated or the effect of different reaction conditions on the kinetics of the amplification reaction can be analyzed. Quantitation of the initial amount of target will first be discussed. Fig. 9 shows the linear relationship between the log of the starting number of template copies and the number of cycles it takes for the amplification in Fig. 8B to reach the arbitrarily chosen fluorescence level of 190. The line shown in Fig. 9 is regression fitted to the data points from 10⁸ to 10³ initial copies (r² > .99). The data point corresponding to 10² initial copies has a statistically significant deviation from this line (zero copies cannot be placed on a logarithmic plot). Besides the data point at 10² copies, the data point most deviant from the regression line is at 10⁵, which, using the equation of the fitted line, predicts a value 13% lower than the known initial number of copies. Similar results are obtained if the fluorescence level chosen in Fig. 8B is anywhere from 125-225.

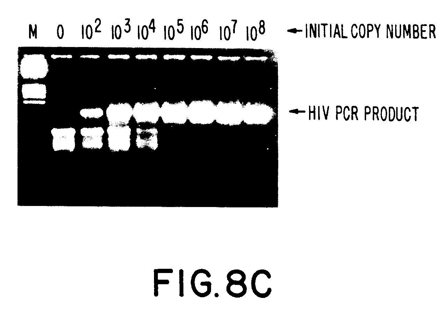

The reason for the deviation from linearity at the data point at 10² copies is shown by the gel electrophoresis analysis of the amplification products in Fig. 8C. The specificity of the amplification is such that, starting with 10⁸ down to 10⁵ copies of template, the only visible product is that of the expected size. When 10⁴ starting copies were used, a smaller, nonspecific amplification product becomes barely visible. This product is more prominent when 10³ copies are used, and when 10² copies are used, an equal amount of nonspecific product and HIV-specific product are made. Only nonspecific product is made in the control with no added template. Since fluorescence due to specific product is indistinguishable from that of nonspecific product, the "baseline" of sensitivity of this assay is at the cycle number by which so much nonspecific product is being synthesized that any additional synthesis of specific product has little effect on the reaction profile. As long as concentration determinations are based on interpolation from a set of standards, i.e., reaction tubes containing known concentrations of starring target nucleic acid sequence, this baseline could be at significantly more cycles than at the cycle number at which the deviation from linearity begins. In practice we have seen that sample-to-sample variation of the reaction profile at less than 10² templates complicates reproducible quantitation below this level (data not shown).

In order to quantitate the initial amount of target nucleic acid in a sample, the normalized fluorescence data from the set of standards are first used to establish the relationship between initial target nucleic acid amount and the number of cycles needed (such as that shown in Fig. 9) to reach an arbitrarily chosen level of fluorescence that is within the range of detection of this instrument. This arbitrary fluorescence value (AFV) is preferably chosen to be in a region of the reaction profile that is, as described with reference to Fig. 8B, parallel among the different standards. This will generally be true if the selected AFV is from 0.1 to 0.5 times the maximum fluorescence value obtained by the standard using the highest initial known target nucleic acid concentration.

After selecting the AFV, for each standard amplification, the correction and normalized fluor value obtained that is nearest the AFV is chosen. Taking this value, and the fluor values for the four cycles preceding and the four cycles following the cycle at which this value occurs, a regression line is fitted that relates cycle number (which can be fractional) to fluorescence. The AFV is then entered into the equation of this regression line and a value for this standard sample of the number of cycles needed to reach the AFV is returned.

Having now determined for each standard the number of cycles needed to reach the AFV, a regression line is fitted to the data that relates the initial target nucleic acid amount to the number of cycles needed to reach the AFV. To now determine the initial target nucleic acid amount of an unknown sample that was amplified together with the standard samples, the number of cycles needed to reach the AFV is determined as was done for the standard sample. This number of cycles (which can be fractional) is entered into the equation of the fitted regression line and the equation returns a value that is the initial amount of target nucleic acid in the unknown sample. This process can be repeated for each unknown sample. The data manipulations just described can easily and rapidly be performed by a microprocessor that is suitably programmed.

This mode of nucleic acid quantitation can be used not only with fluorescence resulting from the non-sequence-specific binding of dyes, but can also be used with fluorescence resulting from the sequence-specific binding of oligonucleotide probes such as those used in the homogenous amplification/detection systems described in U.S. Patent No. 5,210,015. These probes require alternative light sources and filters as described in the above patent. Use of multiple sequence specific probes, each with a distinguishable fluorescence, should allow simultaneous detection and quantitation of multiple nucleic acid target sequences in a single amplification reaction.

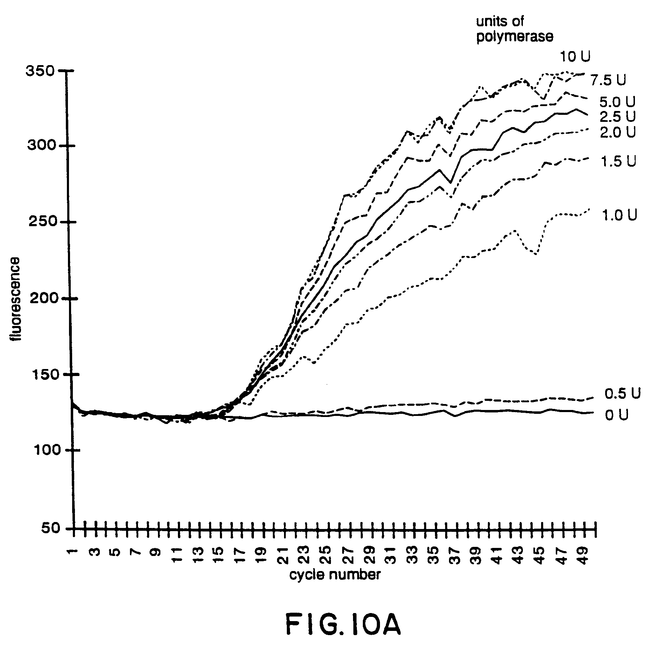

Referring to Figs. 10A-D, analyzing the effect of different reaction conditions on a PCR will be discussed. In general, these effects can be monitored by adding, subtracting, or changing components of a standard PCR and observing the change in the reaction profile. Fig. 10A shows a titration of Taq DNA polymerase. Nine replicate PCRs containing 10⁸ target HIV templates were initiated using a range of Taq polymerase levels as indicated in the figure. For enzyme amounts between 1 and 10 units per reaction, fluorescence begins to be detectable in all reactions by about the same cycle. This indicates that these differences in enzyme levels are having little effect on the efficiency of amplification in early cycles. Differences in the levels of PCR product become apparent in later cycles, however, such that by cycle 50 almost twice as much DNA has been made using 10 units as compared to 1 unit. By gel electrophoresis (not shown), there is no evidence of the production of nonspecific, non-HIV PCR product in these reactions. Using only 0.5 unit Taq polymerase, there is a sudden absence of detectable product. Since PCR is a process in which DNA increases in concentration exponentially, it is perhaps not surprising that there is a threshold level of enzyme below which amplification is an apparent failure.

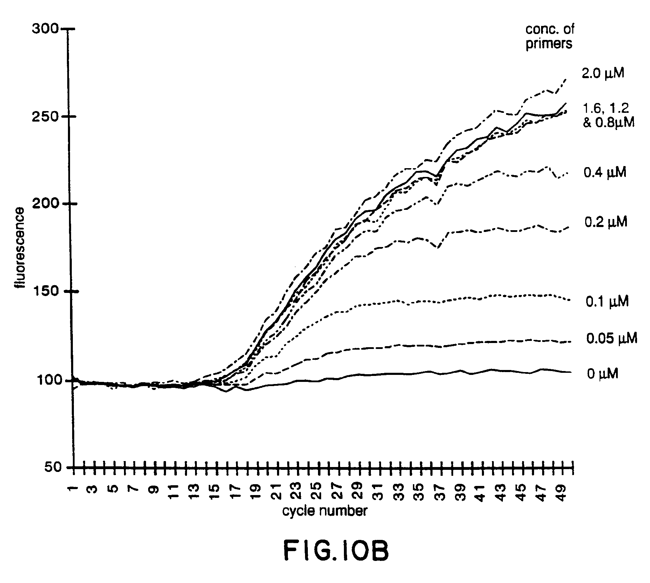

The effect of changing primer concentrations on the kinetics of amplification also can be monitored. Fig. 10B shows profiles of PCRs detecting HIV templates (10⁸ initial ssDNA copies) using primer levels from 2 µM (each primer) down to 0.05 µM. A constant level of enzyme (2.5 U) was used in each PCR. Note that from 0.05 to 0.4 µM in primer concentration, the DNA level in the reaction rises to a certain level and remains constant. If these final product levels are estimated using the data in Fig. 4 and assuming 10.6 pmole/µg of 142 bp PCR product, we see that for 0.05, 0.10, and 0.20 µM primer, 0.05, 0.10, and 0.20 µM product respectively, is made. At these levels, primer concentration is apparently the limiting factor on yield of product DNA. In contrast, at 0.8 µM primer concentration and above, the DNA level is continuing to rise with each thermal cycle, as in Fig. 6A, and increasing amounts of primer have little effect on the profile. By comparison with the data in Fig. 6A, one would conclude that these reactions are now enzyme, and not primer, limited.

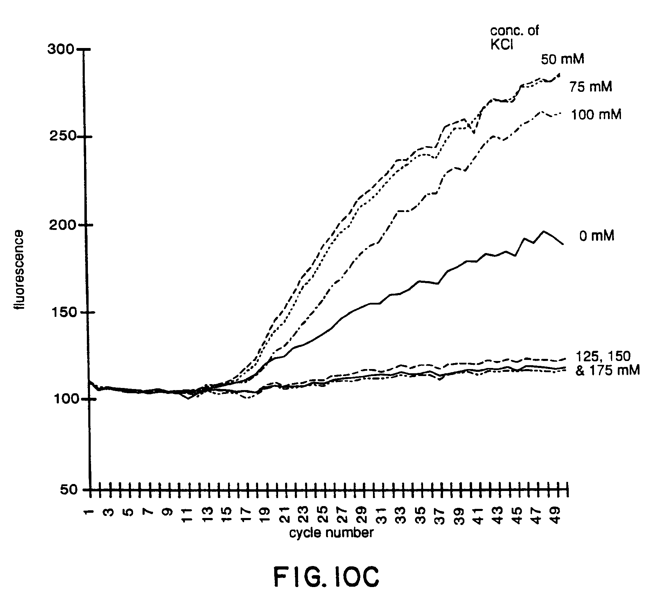

The result of varying KCl concentration using the same HIV test system with 1 µM primers and 2.5 U of enzyme per 100 µl reaction are shown in Fig. 10C. Taq DNA polymerase activity is greatly inhibited by high concentrations of KCl. Consistent with this, at concentrations of KCl of 125 mM or greater there is no detectable PCR product. With activated salmon sperm DNA as template there is an optimum in Taq DNA polymerase activity at 50-90 mM KCl. This assay shows that product yield is highest at 50, 75 and 100 mM KCl with only about half as much being made at 0 mM KCl. It is possible that this difference is due to low ionic strength destabilizing primer annealing, although inconsistent with this is the observation that the efficiency of amplification using 0 mM KCl appears nearly the same as those at 50-100 mM KCl until about cycle 20. Only in later cycles does product yield fall off, similar to the results obtained with less polymerase in Fig. 6A. If primer annealing were destabilized, one would expect that amplification efficiency would be diminished for all cycles.

Lastly, a known inhibitor of PCR, hematin, was added to PCRs at various concentrations. Hematin was added to the same HIV test system used before, except that a primer concentration of 0.2 µM was used. As shown in Fig 10D, at hematin concentrations of 0.2 µM and greater, no detectable DNA product is obtained. At 0.1 µM, the detection of product is delayed by 3-4 cycles, suggesting that, in contrast with the results obtained with 0 mM KCl or reduced enzyme, the efficiency of amplification is being detrimentally affected both in early and later amplification cycles. This difference in reaction profiles suggests that partially inhibited PCRs can be distinguished from uninhibited reactions.

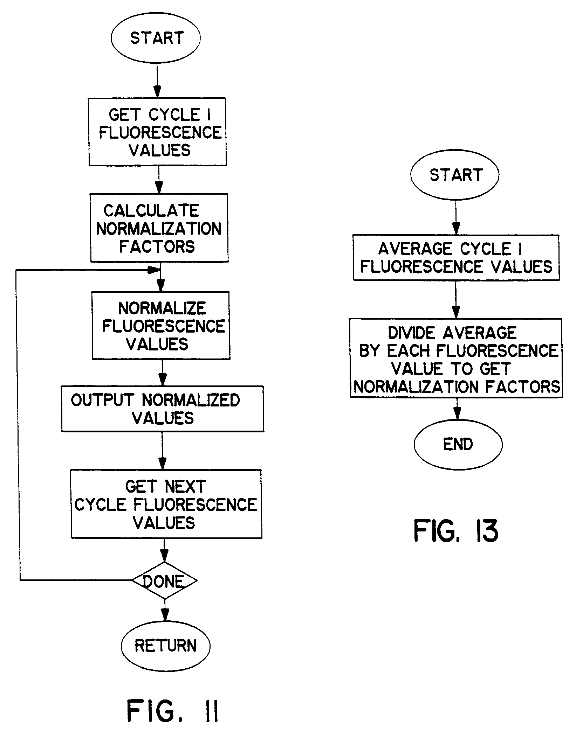

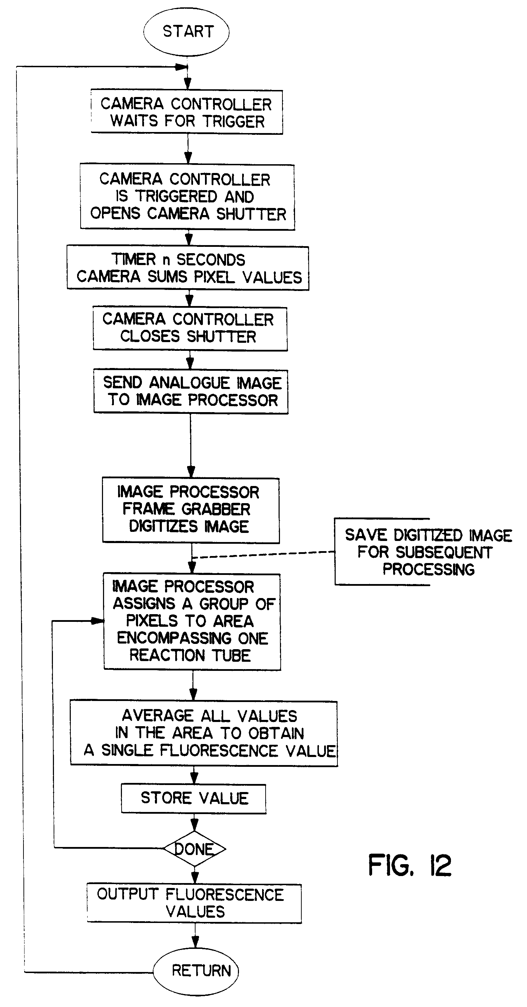

Referring to Figs. 11-13, simplified flow charts illustrating the steps performed by the apparatus of the present invention are shown. Fig. 11 illustrates the overall fluorescence value obtaining steps, while Figs. 12-13 show the fluorescence averaging and normalization subroutines.

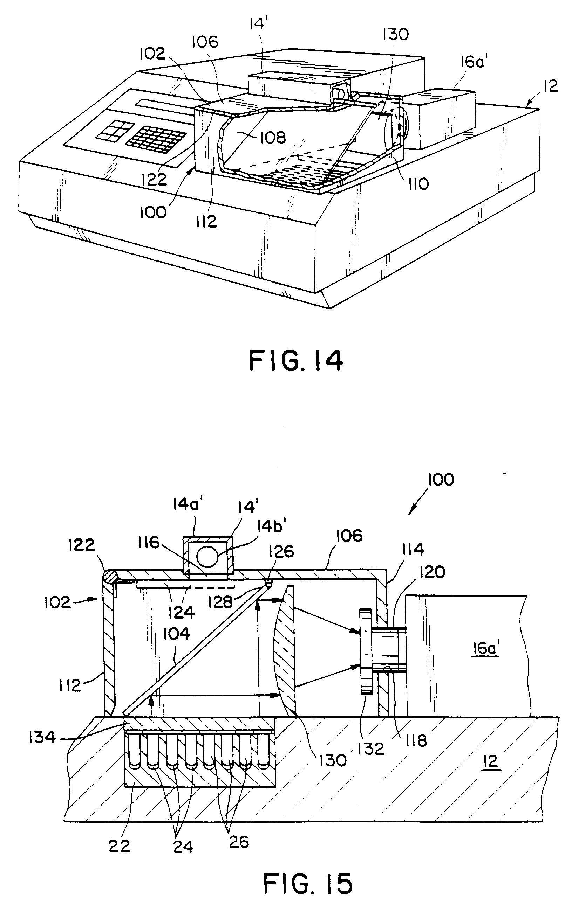

Referring to Fig. 11, the process is started and cycle 1 fluorescence values are obtained. Then, the processor calculates normalization factors for each sample. Then the fluorescence value for each value in that cycle is normalized by multiplying each fluorescence value by its respective normalization factor. The processor then outputs the normalized values and the next cycle of fluorescence values are obtained. This is repeated until the all of the fluorescence images are processed. Referring to Fig. 12, which illustrates how the fluorescence values are obtained, the thermal cycler is started and the excitation light source energized as the camera controller waits for a trigger signal. When the camera controller receives the trigger signal from the processor, it sends a signal to the camera to open the camera shutter for a period of n seconds in which the camera sums pixel values indicative of fluorescence before the shutter is closed. The analog image is sent to the image processor where the image processor frame grabber digitizes the image. At this point the digitized image can be saved for subsequent processing or it can be processed in real-time. In the latter case, the image processor then assigns a group of pixels to an area encompassing one reaction tube and averages all values in that area to obtain a single fluorescence value which is stored. The processor repeats these steps until a single fluorescence value is obtained for all the reaction tubes in that cycle. Then the processor outputs the stored values for that cycle. The foregoing is then repeated for that cycle. Fig. 13 is a subroutine for the normalization factor obtaining step illustrated in Fig. 11. First, the average value for all of the cycle 1 floor values is determined. Then, that average is divided by each fluorescence value in cycle 1 to get normalization factors for each amplification reaction mixture which will then be used for the remaining cycles. Although the flow charts do not include cycle-to-cycle correction, such can also be accommodated by the processor.

Referring to Figs. 14 and 15, a further embodiment of the excitation light and fluorescence emissions sensing arrangement for use in conjunction with apparatus 10 is shown. This excitation and fluorescence sensing arrangement is generally designated with reference numeral 100 and generally comprises a shell or housing 102 for covering recesses 24 in a light-tight manner, a lamp or light source 14' for providing excitation light to reaction samples placed in the recesses, sensor or camera 16a' for detecting or sensing fluorescence emitted from the reaction samples and dichroic mirror 104 for separating the excitation light and fluorescence emissions so that essentially only the emissions are detected by camera 16a'. The fluorescence or amplification data is processed as described above.

Housing or shell 102 is shown as having a top wall 106, side walls 108,110, front wall 112 and rear wall 114. Shell or housing 102 is mounted on the upper surface of thermal cycler 12 such that the housing encloses heat exchanger 22 and, thus, recesses 24, which are formed therein and sized to receive a number of tubes 26, which are configured to hold nucleic acid amplification samples. More specifically, housing 102 is constructed so as to form a light-tight chamber for the samples placed in sample tubes 26 as well as the optical paths between the light source and the samples and the samples and the sensor. Accordingly, the housing is mounted to thermal cycler 12 to provide a light-tight connection therebetween. In addition, except for apertures 116 and 118, which are configured to receive excitation light source or lamp 14' and lens piece 120 of camera 16a', the walls of the shell or housing are imperforate and essentially consist of opaque material. In addition, the walls preferably comprise a material having a relatively low thermal conductivity, such as plastics, to impede heat transfer from the heat conducting member 22 and the samples in tubes 26. One suitable material for the housing walls is polycarbonate.

A mechanism is provided to access the interior of housing or shell 102 and, thus, to the samples in heat exchanger 22. Referring to Figs. 14 and 15, front wall 112 can be hingedly secured to top wall 106 by hinge 122 in order to provide such access. Although a particular configuration has been described with respect to the housing, it should be understood that other configurations can be used to form a light-tight chamber for the excitation and fluorescence emissions light paths as well as the housing for the elements housed in housing 102 which are described in more detail below.

Lamp or light source 14' essentially differs from lamp 14 in configuration. Lamp 14' includes a condensing shield or shroud 14a' and bulb or filament 14b' for emitting light as does lamp 14 and as is conventional in the art. Lamp 14' is selected to provide light having a wavelength corresponding to the wavelength of light needed to excite the fluorescent binding agent present in the sample to be tested. For example, when using ethidium bromide as a fluorescent binding agent, light source 14' preferably is selected to emit UV light at a wavelength of about 302 nm. A suitable light source for providing 302 nm (UV) light is commercially available from U.V. Products, Inc. (CA) under model no. UVM-57 (302 nm). It should be understood, however, that ethidium bromide can be excited at a wavelength of about 480 nm, as well as at about 302 nm. In addition, other fluorophores, such as other DNA binding dyes or the 5' nuclease homogeneous assay probes described above, can be excited by non-UV wavelengths. Thus, a light source that generates 480 nm light can be used in the alternative. However, light sources that generate 480 nm light generally provide a spectrum of visible light. Accordingly, when using such a light source, a filter is used to obtain the desired 480 nm output. Generally, any light source arrangement can be used as long as the desired wavelength is provided to the reaction mixtures. Monochromometers and lasers are examples of suitable light sources.

Lamp 14' is coupled to housing 102 such that the excitation light is directed through aperture 116, which is centered over recesses 24 to minimize nonuniform light distribution thereover, and toward any samples contained in recesses 24. It should be understood that lamp 14' can be positioned at other locations as well. For example, lamp 14' can be completely housed in housing 102 and aperture 116 eliminated. However, the light emitting element, e.g., bulb 14b, is preferably parallel to the upper surface of heat exchanger 22 and centered over the recesses to enhance the uniformity of the light distribution over the heat exchanger for the reasons discussed above. If a filter is used to obtain the desired wavelength, the filter can be mounted, for example, to the housing at aperture 116 between the optical path of the lamp and the samples provided in recesses 24.

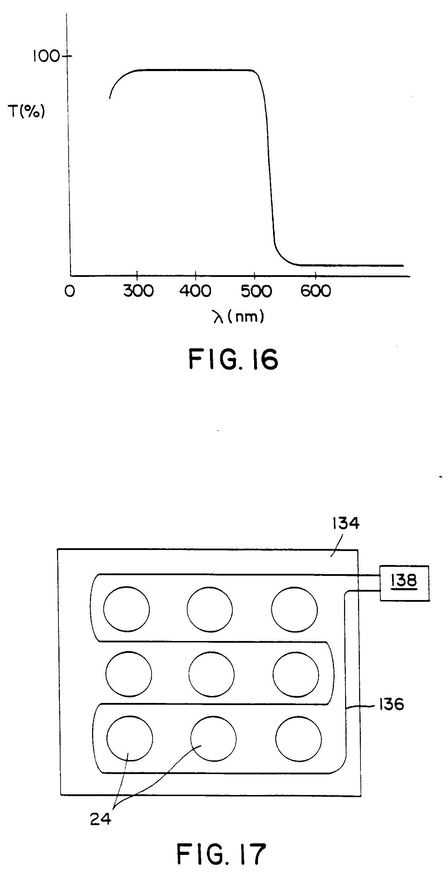

Dichroic mirror 104, which preferably is a low pass dichroic mirror, is positioned directly over heat exchanger 22 and preferably at an angle of about 45° to the upper surface of the heat exchanger to accommodate the orientation of lamp 14' and camera 16a'. In the arrangement shown in the drawings, dichroic mirror 104 is a low pass dichroic mirror, constructed to be transmissive or transparent to light having a wavelength corresponding to that emitted from excitation light source 14' and reflective to light having a wavelength corresponding to that emitted by the samples in recesses 24 when exposed to the excitation light (see Fig. 15). Of course, the transmissive and reflective characteristics of the dichroic mirror are selected according to the particular application. For example, when using ethidium bromide as the fluorescent binding agent, the excitation light wavelength can be about 302 or 480 nm as discussed above. In either case, the emission wavelength is about 600 nm. Thus, the dichroic mirror can be constructed such that it is transmissive to light having a wavelength of about 302 or 480 nm or transmissive to both about 302 and about 480 wavelength light. In both cases the dichroic mirror is constructed to be reflective to light having a wavelength of about 600 nm. Fig. 16 shows a transmissivity v. wavelength curve that is representative of suitable characteristics for the dichroic mirror when using ethidium bromide as a fluorescent binding agent. In this example, the dichroic mirror is highly transmissive to fight having a wavelength in the range of about 300 to 510 nm (the latter being the cut-off wavelength) and then becomes highly reflective as the wavelength approaches 600. It is also noted that a dichroic mirror having the properties illustrated in Fig. 16 can be used when other fluorescent binding agents are used such as fluorescein. Fluorescein has excitation and emission wavelengths of about 495 and 522 nm, respectively. As to the degree of transmissivity and reflectivity, a mirror as shown in Fig. 16 which is capable of transmitting about 85% of the excitation fight, while reflecting about 98% of the emitted fluorescence toward camera 16a' has provided suitable results for detecting nucleic amplification with ethidium bromide in accordance with the present invention.

The following is given for purposes of illustration and is not intended to limit the invention. The specifications of one suitable dichroic mirror for separating excitation and emission light when using ethidium bromide as a fluorescent binding light are as follows: UV Transmitting 510 nm Cut-Off Short Pass: Transmission ≥ 80% Avg. 300-500 nm and ≥ 65% absolute @ 300 nm; reflectivity ≥ 90% abs. 525-610 nm; hard refractary oxide surface coating; substrate - Corning 7940 Fused Silicon; and Chamfer .010 x 45° all edges. Manufacturers of suitable dichroic mirrors include Omega Optical, Inc. (VT)and Janos Optical, Inc. (VT).

Although a low pass arrangement has ben described, a high pass dichroic mirror can be used in the alternative. However, when using a high pass dichroic mirror, the positions of lamp 14' and camera 16a' are reversed as would be apparent to one of ordinary skill.

Returning to the arrangement of the componentry of the embodiment shown in Figs. 14 and 15, dichroic mirror 104 is hingedly secured to ridge or projection 126 by hinge 128 so that the mirror can be pivoted for access to heat exchanger 22. Ridge or projection 126 extends from upper wall 106 and is constructed to prevent or minimize light from light source 14' from passing over hinge 128 toward camera 16a.

A light shutter, diagrammatically shown and designated with reference numeral 124 in Fig. 15, is provided in accordance with the preferred embodiment to limit exposure of the reaction samples to the excitation light. As diagrammatically shown in Fig. 15, shutter 124 can be slidably mounted to upper wall 106 to move between a first position (shown in solid line) where aperture 116 is open and a second position (shown in dotted line) where it covers aperture 116. The shutter can be spring-biased toward the closed position and solenoid actuated to the open position as is conventional in the art. The shutter can be controlled, for example, to open only during the annealing/extension phase of each cycle during PCR, i.e., when maximum fluorescence is expected. In this manner, the shutter improves system performance by increasing sample stability. Since the lamp is closer to the reaction samples in this embodiment, the samples are subjected to relatively strong excitation light intensity, which can cause sample degradation or photo-deactivation, otherwise known as "bleaching." The shutter minimizes the amount of time the sample is exposed to the light and, thus, eliminates or significantly reduces the problem of bleaching. Although the shutter is shown coupled to housing 102, it can be coupled to lamp 14', positioned directly above the heat conducting block and, thus, the samples or in some other manner to control sample exposure to the excitation light. Other mechanisms to control or time such exposure can also be used.

A field or plano convex lens 130 is provided between dichroic mirror 104 and lens piece 120 of camera 16a'. Field lens 130 deviates the rays reflected from mirror 104 inward so that essentially all of the fluorescence emitted from the samples and heat exchanger 22 is directed toward lens piece 120. Field lens 130 preferably is slidably mounted to housing 102 so that it can be moved toward or away from lens piece 120 depending on the wavelength of the sample emissions as would be apparent to one of ordinary skill.

A single filter or a plurality of filters, such as a filter wheel, can be coupled to the camera to permit only certain wavelength(s) to be detected. Referring to Fig. 15, a filter wheel 132 is shown coupled to lens piece 120, as is conventional in the art, so that multiple labels or targets can be detected and monitored. That is, the filters can be interchanged by rotating the wheel so that detection is limited to the desired wavelength. The interchanging step can be carried out during each annealing/extension phase or after a preselected number of cycles depending on the application. Alternatively, it is contemplated to use a camera that distinguishes wavelengths such as a spectral imager.

Referring to Fig. 15, a heated transparent or transmissive window or member 134 preferably is provided to improve thermal cycling conditions. Member 134 should be at least sufficiently transmissive so as to allow for the effective excitation of the samples and detection of the accompanying fluorescence. Heating member or window 134 can comprise quartz, for example, and can be placed on reaction tubes 26 or slightly thereabove. Heating member 134 generally is provided to maintain the thermal cycling temperature profiles of the reaction mixtures to follow the preselected profiles as close as possible and minimize reflux. The heating member is preferably maintained at a temperature within the range of about 95-105°C, and preferably about 100°C. That is, the window is preferably maintained at a temperature corresponding to the denaturation temperature. Member 134 can be heated with nichrome wire, for example, or other heating elements according to conventional practices.

Referring to Fig. 17, a heating member using a nichrome wire heating element is shown. It is also noted that although heating element 134 is shown as covering a heat exchanger having only 9 recesses, this is merely for purposes of simplifying the drawing. Nichrome wire 136 can be embedded in member 134 or secured to the surface thereof. The wire preferably is arranged not to extend over the recesses 24 in the heat exchanger and, thus, not to interfere with the optical paths of the excitation and emission light. The wire also is coupled to a conventional temperature controller 138 that maintains the temperature of element 134 at the desired temperature.

A display preferably is coupled to the processor for displaying data such as indicating if the target sequence is present as well as its concentration. In addition, with the continuous monitoring of the amplifications (discussed above), means for displaying the detected amplifications can be displayed as the amplifications are progressing. This enables reductions in thermal cycling times. For example, if a decision can be made that a reaction mixture contains the target sequence at a relatively early stage, the sample can be removed and another placed in its position. Throughput is thereby increased. This is especially advantageous when loading a heat exchanger with up to 96 wells with different reaction mixtures (e.g., some testing for a genetic disease, some testing for HIV and so forth) or when the mixtures are loaded at different times.

The above is a detailed description of a particular embodiment of the invention. It is recognized that departures from the disclosed embodiment may be made within the scope of the invention and that obvious modifications will occur to a person skilled in the art. The full scope of the invention is set out in the claims that follow and their equivalents. Accordingly, the claims and specification should not be construed to unduly narrow the full scope of protection to which the invention is entitled.

## Claims

1. An apparatus for simultaneously monitoring multiple nucleic acid amplifications characterized in that it comprises:
(a) a thermal cycler (12) including a heat conducting member (22) having multiple recesses (24) formed therein;
(b) a light source (14,14') optically coupled to the thermal cycler and arranged to distribute light over a plurality of the recesses in the heat conducting member; and
(c) a sensor (16,16a') arranged for simultaneously detecting light emitted from the plurality of recesses.

2. An apparatus according to claim 1 characterized in that it further comprises means for generating an average value for light detected by the sensor with respect to each light emitting recess.

3. An apparatus according to claim 1 characterized in that it further comprises
(a) means for cycling the temperature of the heat conducting member according to a preselected temperature versus time profile for multiple cycles;
(b) multiple nucleic acid amplification reaction mixtures, each mixture comprising a fluorescent binding agent and being disposed in a different one of the recesses;
and in that
the light source provides an essentially uniform light flux to a plurality of the reaction mixtures; and
the sensor being optically coupled to the plurality of reaction mixtures for simultaneously detecting fluorescence emitted from each of the plurality of reaction mixtures.

4. An apparatus according to claim 3 characterized in that it further comprises means for generating an average fluorescence intensity value for each excited reaction mixture based on the detected fluorescence therefor.

5. An apparatus according to claim 4 characterized in that it further comprises means for generating normalized fluorescence intensity values from the average fluorescence intensity values.

6. An apparatus according to claim 1 characterized in that
the recesses of the heat conducting member of the thermal cycler are formed through a surface thereof for receiving reaction vessels (26) containing a nucleic acid amplification reaction mixtures; and in that
said sensor is an imaging device optically coupled to the heat conducting member for generating an image of the surface and reaction vessels when the vessels are disposed in the recesses of the heat conducting member.

7. An apparatus according to claim 6 characterized in that it further comprises a light source arranged to provide light over said heat conducting member surface.

8. An apparatus according to claim 7 wherein said light source is arranged to distribute light essentially uniformly along said heat conducting member surface.

9. An apparatus according to claim 7 wherein said light source comprises a UV lamp.

10. An apparatus according to claim 6 wherein said imaging device comprises a CCD-imaging array.

11. An apparatus according to claim 1 characterized in that the heat conducting member of the thermal cycler has multiple recesses formed therein and adapted for receiving nucleic acid amplification reaction mixtures, and in that said apparatus further comprises
a housing positioned over the heat conducting member and coupled to the thermal cycler;
the light source is arranged to emit light in the housing and toward a plurality of the recesses; and
a dichroic mirror positioned in the housing and optically coupled to the light source and to the plurality of recesses, the dichroic mirror being transmissive to light having a first predetermined wavelength and reflective to light having a second predetermined wavelength that differs from the first predetermined wavelength; and
the sensor being optically coupled to the dichroic mirror and arranged for simultaneously detecting light emitted from the plurality of the recesses.

12. An apparatus according to claim 11 wherein the first predetermined wavelength corresponds to the wavelength of light generated by the light source and the second predetermined wavelength corresponds to the wavelength of light emitted from a nucleic acid amplification mixture when that mixture is disposed in one of the recesses and exposed to light from the light source.

13. An apparatus according to claim 11 wherein the first predetermined wavelength is about 200-550 nm.

14. An apparatus according to claim 11 wherein the housing comprises opaque material and is constructed to form a light-tight chamber in which the dichroic mirror and the sensor are arranged.

15. An apparatus according to claim 11 wherein the heat conducting member has an upper surface and the dichroic mirror forms an angle of about 45° with the upper surface.

16. An apparatus according to claim 11 wherein said sensor comprises a charge coupled device.

17. An apparatus according to claim 11 further comprising a shutter for regulating the exposure of the recesses to the light source at preselected intervals.

18. An apparatus according to claim 11 further comprising a window comprising transparent material and a heating element coupled thereto, the window being disposed between the recesses and the dichroic mirror.

19. An apparatus according to claim 11 further comprising a field lens arranged between the dichroic mirror and the sensor.

20. An apparatus according to claim 11 further comprising a filter wheel coupled to the sensor.

21. An apparatus according to claim 1 characterized in that the heat conducting member of the thermal cycler has multiple recesses formed therein for receiving nucleic acid amplification reaction mixtures including a nucleic acid sequence and a fluorescent binding agent and in that said apparatus further comprises
a housing positioned over the heat conducting member and being coupled to the thermal cycler; whereby
the light source is arranged to emit light in the housing; and
the sensor is arranged in the housing and
a dichroic mirror positioned in the housing and above the recesses, the dichroic mirror being transmissive to light having a wavelength corresponding to the wavelength of light generated by the light source and reflective to light having a wavelength corresponding to the wavelength of fluorescence emitted from a nucleic acid amplification mixture, including a fluorescent binding agent, when that mixture is disposed in one of the recesses formed in the heat conducting member and exposed to light from the light source, the mirror be oriented to form an optical path between the recesses and the sensor.

22. An apparatus according to claim 21 wherein said dichroic mirror comprises a first surface and a second surface, the first surface generally facing the light source and the second surface generally facing the recesses and the sensor.

## Patentansprüche

1. Vorrichtung zur gleichzeitigen Überwachung mehrerer Nukleinsäure-Amplifikationen, gekennzeichnet durch:
(a) einen Thermalcycler (12) mit einem Wärmeleiter (22), in welchem eine Anzahl Ausnehmungen (24) gebildet sind,
(b) eine mit dem Thermalcycler optisch gekoppelte Lichtquelle (14, 14') zur Abgabe von Licht auf die Anzahl Ausnehmungen im Wärmeleiter, und
(c) einen Sensor (16, 16a') zur gleichzeitigen Erfassung des aus der Anzahl Ausnehmungen austretenden Lichts.

2. Vorrichtung nach Anspruch 1, dadurch gekennzeichnet, dass sie ausserdem Mittel zur Bildung eines Mittelwerts des durch den Sensor für jede lichtemittierende Ausnehmung erfassten Lichts enthalt.

3. Vorrichtung nach Anspruch 1, dadurch gekennzeichnet, dass sie ausserdem
(a) Mittel zur Durchführung von Temperaturzyklen des Wärmeleiters gemäss einem vorbestimmten Temperatur-Zeit-Profil für mehrere Zyklen aufweist, sowie
(b) eine Vielzahl Reaktionsgemische für Nukleinsäure-Amplifikationen enthalten, wobei jedes Reaktionsgemisch ein fluoreszierendes Bindemittel enthält und in je einer Ausnehmung angeordnet ist;
und dass
die Lichtquelle die Abgabe eines Lichtstromes mit im wesentlichen gleichmässigen Lichtintensität an die Vielzahl Reaktionsgemische gestattet und
der Sensor mit der Anzahl Reaktionsgemische optisch gekoppelt ist, um die jeweils von den Reaktionsgemischen abgegebene Fluoreszenz gleichzeitig zu erfassen.

4. Vorrichtung nach Anspruch 3, weiterhin gekennzeichnet durch Mittel zur Bildung eines Mittelwerts der Fluoreszenz-Intensität jedes angeregten Reaktionsgemischs anhand der erfassten Fluoreszenz desselben.

5. Vorrichtung nach Anspruch 4, weiterhin gekennzeichnet durch Mittel zur Bildung standardisierter Fluoreszenz-Intensitätswerte anhand der Fluoreszenz-Intensitätsmittelwerte.

6. Vorrichtung nach Anspruch 1, dadurch gekennzeichnet, dass die Ausnehmungen des Wärmeleiters des Thermalcyclers in einer Oberfläche desselben ausgebildet sind, um Reaktionsgefässe aufnehmen, welche Reaktionsgemische für Nukleinsäure-Amplifikationen enthalten, und dass
der Sensor eine Abbildungsvorrichtung ist, welche mit dem Wärmeleiter optisch gekoppelt ist, um eine Abbildung der Oberfläche und der Reaktionsgefässe zu erzeugen, wenn sich die Gefässe in den Ausnehmungen des Wärmeleiters befinden.

7. Vorrichtung nach Anspruch 6, weiterhin gekennzeichnet durch eine Lichtquelle zur Beleuchtung der Oberfläche des Wärmeleiters.

8. Vorrichtung nach Anspruch 7, dadurch gekennzeichnet, dass die Lichtquelle geeignet ist, das Licht im wesentlichen gleichmässig entlang der Oberfläche des Wärmeleiters zu verteilen.

9. Vorrichtung nach Anspruch 7, dadurch gekennzeichnet, dass die Lichtquelle eine UV-Lampe aufweist.

10. Vorrichtung nach Anspruch 6, dadurch gekennzeichnet, dass die Abbildungsvorrichtung eine CCD-Abbildungsanordnung aufweist.

11. Vorrichtung nach Anspruch 1, dadurch gekennzeichnet, dass im Wärmeleiter des Thermalcyclers mehrere Ausnehmungen zur Aufnahme von Reaktionsgemischen für Nukleinsäure-Amplifikationen gebildet sind, und die Vorrichtung weiterhin
ein über dem Wärmeleiter angeordnetes Gehäuse aufweist, welches mit dem Thermalcycler verbunden ist und
die Lichtquelle im Gehäuse Licht aussendet und eine Anzahl Ausnehmungen beleuchtet, sowie
ein dichroitischer Spiegel im Gehäuse angeordnet ist, der mit der Lichtquelle und der Anzahl Ausnehmungen optisch gekoppelt ist, wobei der dichroitische Spiegel für Licht mit einer ersten vorbestimmten Wellenlänge durchlässig ist und Licht mit einer zweiten vorbestimmten, von der ersten vorbestimmten Wellenlänge abweichenden Wellenlänge reflektiert, und
der Sensor mit dem dichroitischen Spiegel optisch gekoppelt ist und das von der Anzahl Ausnehmungen abgegebene Licht gleichzeitig erfassen kann.

12. Vorrichtung nach Anspruch 11, dadurch gekennzeichnet, dass die erste vorbestimmte Wellenlänge der Wellenlänge des von der Lichtquelle erzeugten Lichts entspricht und die zweite vorbestimmte Wellenlänge der Wellenlänge des Lichts entspricht, welches von einem Nukleinsäure-Amplifikationsgemisch ausgeht, wenn sich das Gemisch in einer der Ausnehmungen befindet und von der Lichtquelle beleuchtet wird.

13. Vorrichtung nach Anspruch 11, dadurch gekennzeichnet, dass die erste vorbestimmte Wellenlänge ungefähr 200 - 550 nm beträgt.

14. Vorrichtung nach Anspruch 11, dadurch gekennzeichnet, dass das Gehäuse undurchsichtiges Material aufweist und als lichtdichte Kammer ausgebildet ist, in welcher der dichroitische Spiegel und der Sensor angeordnet sind.

15. Vorrichtung nach Anspruch 11, dadurch gekennzeichnet, dass der Wärmeleiter eine obere Fläche aufweist und der dichroitische Spiegel einen Winkel von ungefähr 45° zu der oberen Fläche bildet.

16. Vorrichtung nach Anspruch 11, dadurch gekennzeichnet, dass der Sensor eine ladungsgekoppelte Vorrichtung aufweist.

17. Vorrichtung nach Anspruch 11, weiterhin gekennzeichnet durch eine Blende zur Regulierung der Beleuchtung der Ausnehmungen durch die Lichtquelle in bestimmten Zeitabständen.

18. Vorrichtung nach Anspruch 11, weiterhin gekennzeichnet durch ein Fenster aus durchsichtigem Material und ein damit gekoppeltes Heizelement, wobei das Fenster zwischen den Ausnehmungen und dem dichroitischen Spiegel angeordnet ist.

19. Vorrichtung nach Anspruch 11, weiterhin gekennzeichnet durch eine Feldlinse, welche zwischen dem dichroitischen Spiegel und dem Sensor angeordnet ist.

20. Vorrichtung nach Anspruch 11, weiterhin gekennzeichnet durch einen mit dem Sensor gekoppelten Filterrevolver.

21. Vorrichtung nach Anspruch 1, dadurch gekennzeichnet, dass im Wärmeleiter des Thermalcyclers mehrere Ausnehmungen zur Aufnahme von Reaktionsgemischen für Nukleinsäure-Amplifikationen mit einer Nukleinsäuresequenz und einem fluoreszierenden Bindemittel gebildet sind, und dass die Vorrichtung weiterhin
ein über dem Wärmeleiter angeordnetes Gehäuse aufweist, welches mit dem Thermalcycler verbunden ist, wobei
die Lichtquelle im Gehäuse Licht aussendet und
der Sensor im Gehäuse angeordnet ist, sowie
einen dichroitischen Spiegel, der im Gehäuse und über den Ausnehmungen angeordnet ist, wobei der dichroitische Spiegel für Licht mit einer der Wellenlänge durchlässig ist, welche der Wellenlänge des von der Lichtquelle erzeugten Lichts entspricht und Licht mit einer zweiten Wellenlänge reflektiert, welche der Wellenlänge der Fluoreszenz entspricht, welche von einem Nukleinsäure-Amplifikationsgemisch mit einem fluoreszierenden Bindemittel ausgeht, wenn sich das Gemisch in einer der im Wärmeleiter ausgebildeten Ausnehmungen befindet und von der Lichtquelle beleuchtet wird, wobei der Spiegel so ausgerichtet ist, dass ein optischer Weg zwischen den Ausnehmungen und dem Sensor gebildet wird.

22. Vorrichtung nach Anspruch 21, dadurch gekennzeichnet, dass der dichroitische Spiegel eine erste und eine zweite Oberfläche aufweist, wobei die erste Oberfläche allgemein der Lichtquelle zugewandt und die zweite Oberfläche allgemein den Ausnehmungen und dem Sensor zugewandt ist.

## Revendications

1. Appareil pour la surveillance simultanée d'amplifications multiples d'acides nucléiques, caractérisé en ce qu'il comprend:
(a) un cycleur thermique (12) comprenant un élément conducteur de chaleur (22) dans lequel est formée une pluralité d'évidements (24),
(b) une source de lumière (14, 14') optiquement accouplée au cycleur thermique et capable de distribuer la lumière sur une pluralité d'évidements de l'élément conducteur de chaleur, et
(c) un détecteur (16, 16a') capable de détecter simultanément la lumière émise de ladite pluralité d'évidements.

2. Appareil selon la revendication 1, caractérisé en ce qu'il comprend en outre des moyens permettant de générer une valeur moyenne de la lumière détectée par le détecteur pour chaque évidement émetteur de lumière.

3. Appareil selon la revendication 1, caractérisé en ce qu'il comprend en outre
(a) des moyens permettant d'effectuer des cycles thermiques de l'élément conducteur de chaleur selon un profil température/temps déterminé pour plusieurs cycles,
(b) une pluralité des mélanges réactionnels pour amplifications d'acides nucléiques, chaque mélange comprenant un liant fluorescent et étant disposé dans un évidement différent,
et en ce que
la source de lumière fournit un flux lumineux essentiellement uniforme vers une pluralité desdits mélanges réactionnels,
le détecteur étant optiquement accouplé à ladite pluralité de mélanges réactionnels afin de détecter simultanément la fluorescence émise par chacun parmi la pluralité de mélanges réactionnels.

4. Appareil selon la revendication 1, caractérisé en ce qu'il comprend en outre des moyens permettant de générer une valeur moyenne de l'intensité de la fluorescence pour chaque mélange réactionnel excité sur la base de la fluorescence détectée de ce dernier.

5. Appareil selon la revendication 1, caractérisé en ce qu'il comprend en outre des moyens permettant de générer des valeurs standardisées de l'intensité de la fluorescence sur la base des valeurs moyennes de l'intensité de la fluorescence.

6. Appareil selon la revendication 1, caractérisé en ce que les évidements de l'élément conducteur de chaleur du cycleur thermique sont formés dans une surface de ce dernier pour recevoir des réacteurs (26) contenant des mélanges réactionnels pour amplifications d'acides nucléiques, et en ce que
ledit détecteur est un dispositif imageur optiquement accouplé à l'élément conducteur de chaleur pour générer une image de la surface et des réacteurs lorsque les réacteurs sont disposés dans les évidements de l'élément conducteur de chaleur.

7. Appareil selon la revendication 6, caractérisé en ce qu'il comprend en outre une source de lumière permettant d'illuminer ladite surface de l'élément conducteur de chaleur.

8. Appareil selon la revendication 7, caractérisé en ce que ladite source de lumière permet de distribuer la lumière essentiellement uniformément le long de ladite surface de l'élément conducteur de chaleur.

9. Appareil selon la revendication 7, caractérisé en ce que ladite source de lumière comprend une lampe à rayons UV.

10. Appareil selon la revendication 6, caractérisé en ce que ledit dispositif imageur comprend un imageur à mosaïque C.C.D.

11. Appareil selon la revendication 1, caractérisé en ce que l'élément conducteur de chaleur du cycleur thermique a une pluralité d'évidements formés dans ledit élément et adaptés à recevoir des mélanges réactionnels pour amplifications d'acides nucléiques, et en ce que ledit appareil comprend en outre
un boîtier disposé sur l'élément conducteur de chaleur et relié au cycleur thermique, et que
la source de lumière permet l'émission de lumière dans le boîtier et vers une pluralité desdits évidements, et
un miroir dichroïque situé dans le boîtier et optiquement accouplé à la source de lumière et à ladite pluralité d'évidements, le miroir dichroïque étant transmissif de lumière ayant une première longueur d'ondes prédéterminée et réfléctif de lumière ayant une deuxième longueur d'ondes prédéterminée qui est différente de la première longueur d'ondes prédéterminée, et
le détecteur étant optiquement accouplé au miroir dichroïque et capable de détecter simultanément la lumière émise de la pluralité d'évidements.

12. Appareil selon la revendication 11, caractérisé en ce que la première longueur d'ondes prédéterminée correspond à la longueur d'ondes de la lumière générée par la source de lumière, et la deuxième longueur d'ondes prédéterminée correspond à la longueur d'ondes de la lumière émise par un mélange pour amplification d'acides nucléiques lorsque ce mélange est disposé dans l'un des évidements et exposé à la lumière de la source de lumière.

13. Appareil selon la revendication 11, caractérisé en ce que la première longueur d'ondes prédéterminée est égale à environ 200 à 550 nm.

14. Appareil selon la revendication 11, caractérisé en ce le boîtier comporte de la matière opaque et est construit sous forme d'une chambre étanche à la lumière dans laquelle sont disposés le miroir dichroïque et le détecteur.

15. Appareil selon la revendication 11, caractérisé en ce que l'élément conducteur de chaleur présente une face supérieure, et que le miroir dichroïque forme un angle d'environ 45° avec ladite face supérieure.

16. Appareil selon la revendication 11, caractérisé en ce que ledit détecteur comprend un dispositif à couplage de charge.

17. Appareil selon la revendication 11, caractérisé en ce qu'il comprend en outre un diaphragme permettant de régler l'exposition des évidements à la source de lumière à des intervalles prédéterminés.

18. Appareil selon la revendication 11, caractérisé en ce qu'il comprend en outre une fenêtre comportant de la matière transparente et un élément de chauffage y relié, la fenêtre étant disposée entre les évidements et le miroir dichroïque.

19. Appareil selon la revendication 11, caractérisé en ce qu'il comprend en outre une lentille de champ disposée entre le miroir dichroïque et le détecteur.

20. Appareil selon la revendication 11, caractérisé en ce qu'il comprend en outre un revolver de filtres relié au détecteur.

21. Appareil selon la revendication 1, caractérisé en ce que des évidements multiples sont formés dans l'élément conducteur de chaleur du cycleur thermique pour recevoir des mélanges réactionnels pour amplifications d'acides nucléiques contenant une séquence d'acide nucléique et un liant fluorescent, et en ce que ledit appareil comprend en outre
un boîtier disposé sur l'élément conducteur de chaleur et relié au cycleur thermique,
la source de lumière permettant l'émission de lumière dans le boîtier, et
le détecteur étant disposé dans le boîtier, et
un miroir dichroïque situé dans le boîtier et au-dessus des évidements, le miroir dichroïque étant transmissif de lumière ayant une longueur d'ondes qui correspond à la longueur d'ondes de la lumière générée par la source de lumière et réfléctif de lumière ayant une longueur d'ondes qui correspond à la longueur d'ondes de la fluorescence de la lumière émise par un mélange pour amplification d'acide nucléique contenant un liant fluorescent, lorsque ce mélange est disposé dans l'un des évidements formés dans l'élément conducteur de chaleur et exposé à la lumière de la source de lumière, le miroir étant orienté de manière à former un parcours optique entre les évidements et le détecteur.

22. Appareil selon la revendication 21, caractérisé en ce que ledit miroir dichroïque comprend une première surface et une deuxième surface, la première surface étant généralement en regard de la source de lumière, et la deuxième surface étant généralement en regard des évidements et du détecteur.
